# EUROPEAN PATENT APPLICATION

(11) **EP 4 218 771 A1**
(43) Date of publication of application: **02.08.2023**
(21) Application number: 22199393.4
(22) Date of filing: 27.03.2016
(51) Int. Cl.: A61K 31/7105, A61K 45/06, C12N 15/113, A61P 25/00

(54) **METHODS OF TREATING MOTOR NEURON DISEASES**

(30) Priority: 27.03.2015 US 201562139568 P
(62) Divisional of application: 16771532.5
(71) Applicant: Yeda Research and Development Co. Ltd, 7610002 Rehovot (IL)
(72) Inventor: HORNSTEIN, Eran, 7630243 Rehovot (IL); REICHENSTEIN, Irit, 7610002 Rehovot (IL); CHEN, Alon, 7610002 Rehovot (IL)
(74) Representative: Dennemeyer & Associates S.A.

(57) **Abstract**

The present invention refers to an agent selected from the group consisting of miR-218, miR-218*, precursor thereof and a polynucleotide sequence encoding miR-218 or miR-218* or precursor thereof, for use in the treatment of a motor neuron disease (MND).

## Description

### FIELD AND BACKGROUND OF THE INVENTION

The present invention, in some embodiments thereof, relates to methods of treating motor neuron diseases and, more particularly, but not exclusively, to Amyotrophic Lateral Sclerosis (ALS).

Motor neuron diseases (MND) belong to a group of neurological disorders attributed to the destruction of motor neurons of the central nervous system and degenerative changes in the motor neuron pathway. Such diseases are different from other neurodegenerative diseases including Parkinson's disease, Alzheimer's disease, olivopontocerebellar atrophy, etc., which are caused by the destruction of neurons other than motor neurons. Typically, MNDs are progressive, degenerative disorders that affect nerves in the upper or lower parts of the body. Generally, MNDs strike in middle age. Symptoms may include difficulty swallowing, limb weakness, slurred speech, impaired gait, facial weakness and muscle cramps. Respiration may be affected in the later stages of these diseases. The cause(s) of most MNDs are not known, but environmental, toxic, viral or genetic factors are all suspects.

Motor neurons, including upper motor neurons and lower motor neurons, affect voluntary muscles, stimulating them to contract. Upper motor neurons originate in the cerebral cortex and send fibers through the brainstem and the spinal cord, and are involved in controlling lower motor neurons. Lower motor neurons are located in the brainstem and the spinal cord and send fibers out to muscles. Lower motor neuron diseases are diseases involving lower motor neuron degeneration. When a lower motor neuron degenerates, the muscle fibers it normally activates become disconnected and do not contract, causing muscle weakness and diminished reflexes. Loss of either type of neurons results in weakness, muscle atrophy (wasting) and painless weakness are the clinical hallmarks of MND.

Amyotrophic Lateral Sclerosis (ALS) is a fatal motor neuron disease characterized by a loss of pyramidal cells in the cerebral motor cortex (i.e., giant Betz cells), anterior spinal motor neurons and brain stem motor neurons, and degeneration thereof into pyramidal cells. ALS shows, from a clinical aspect, both upper motor neurons and lower motor neurons signs, and shows rapid clinical deterioration after onset of the disease, thus leading to death within a few years.

Micro-RNAs (also known as miRNAs) are 20- to 24-nucleotide (nt) RNA molecule members of the family of non-coding small RNAs. Micro-RNAs were identified in mammals, worms, fruit flies and plants and are believed to regulate the stability of their target messenger RNA (mRNA) transcripts in a tissue- and cell type-specific manner. Principally, micro-RNAs regulate RNA stability by either binding to the 3'-untranslated region (3'-UTR) of target mRNAs and thereby suppressing translation, or in similar manner to siRNAs, binding to and destroying target transcripts in a sequence-dependent manner.

Micro-RNAs have been implicated in various neurological diseases such as Fragile X syndrome, spinal muscular atrophy (SMA), early onset parkinsonism (Waisman syndrome) and X-linked mental retardation (MRX3), as well as various cancers and precancerous conditions such as Wilm's tumor, testicular germ cell tumor, chronic lymphocytic leukemia (CLL), B cell leukemia, precancerous and neoplastic colorectal tissues and Burkkit's lymphoma [reviewed in Gong H, et al., 2004, Medical Research Reviews, Published online in Wiley InterScience (wwwdotintersciencedotwileydotcom)].

U.S. Patent Application Publication No. 20060247193 teaches administration of over 100 miRNAs for the treatment of MNDs including ALS.

U.S. Patent Application Publication No. 20090246136 teaches administration of miR-206 and/or miR-1 for the treatment of MNDs including ALS.

Figlewicz et al [Human Molecular Genetics, Volume 3, 1994] teaches that variants of the heavy neurofilament subunit are associated with the development of ALS.

Additional relevant background art includes U.S. Patent Application Publication No. 20080176766, WO2013124816, WO2010064248, and WO2014/020608.

### SUMMARY OF THE INVENTION

According to an aspect of the invention there is provided a use of an agent selected from the group consisting of miR-218, miR-218*, precursor thereof and a polynucleotide sequence encoding miR-218 or miR-218* or precursor thereof, for the preparation of a medicament for the treatment of a motor neuron disease (MND).

According to an aspect of the invention there is provided a method of treating a subject having MND, the method comprising administering to the subject a therapeutically effective amount of an agent selected from the group consisting of miR-218, miR-218*, precursor thereof and a polynucleotide sequence encoding miR-218 or miR-218* or precursor thereof, thereby treating the MND in the subject.

According to an aspect of the invention there is provided a use of an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, for the preparation of a medicament for the treatment of MND.

According to an aspect of the invention there is provided a method of treating a subject having MND, the method comprising administering to the subject a therapeutically effective amount of an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, thereby treating the MND in the subject.

According to an aspect of the invention there is provided an article of manufacture comprising an agent selected from the group consisting of miR-218, miR-218*, precursor thereof and a polynucleotide sequence encoding miR-218 or miR-218* or precursor thereof, and an additional anti-MND agent, being packaged in a packaging material and identified in print, in or on the packaging material for use in the treatment of MND.

According to an aspect of the invention there is provided an article of manufacture comprising an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, thereby treating the MND, and an additional anti-MND agent, being packaged in a packaging material and identified in print, in or on the packaging material for use in the treatment of MND.

According to embodiments of the invention, the administering is effected into the CNS of the subject.

According to embodiments of the invention, the agent is formulated for administration into the CNS of a subject in need thereof.

According to embodiments of the invention, the additional anti-MND agent is an anti-ALS agent.

According to embodiments of the invention, the MND is selected from the group consisting of Amyotrophic Lateral Sclerosis (ALS), primary lateral sclerosis, progressive muscular atrophy, pseudobulbar palsy, progressive bulbar palsy, lower motor neuron disease, spinal muscular atrophy and epilepsy.

According to embodiments of the invention, the MND comprises Amyotrophic Lateral Sclerosis (ALS).

According to embodiments of the invention, the ALS is an inherited ALS.

According to embodiments of the invention, the ALS is a sporadic ALS.

According to embodiments of the invention, the subject is a human subject.

According to embodiments of the invention, the miR-218 is as set forth in SEQ ID NO: 3.

According to embodiments of the invention, the miR-218* is as set forth in SEQ ID NO: 4 or 5.

According to embodiments of the invention, the agent comprises a polynucleotide.

According to embodiments of the invention, the agent comprises an agent for genome editing.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

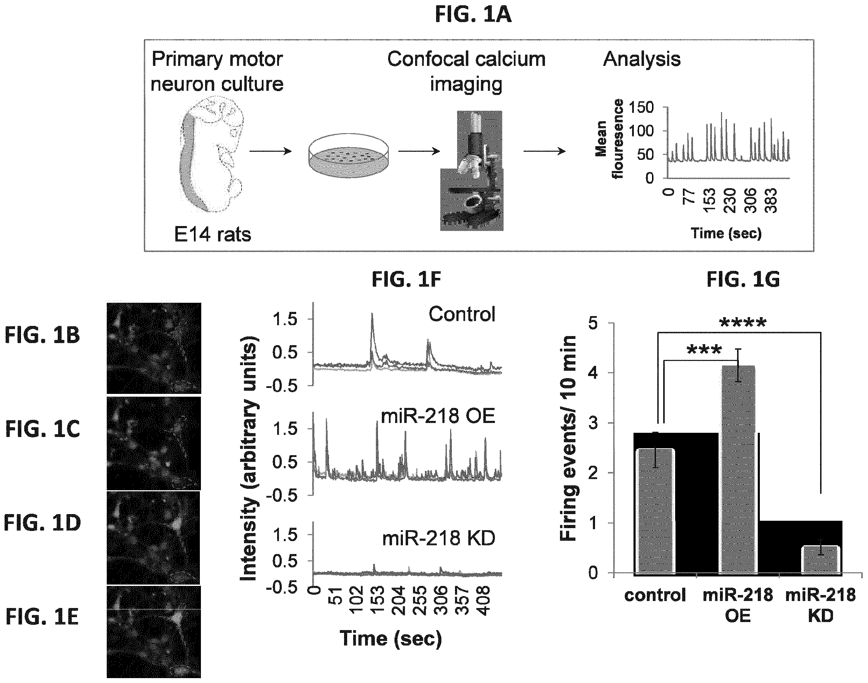

FIGS. 1A-G illustrate that miR-218 controls neuronal excitability. Primary spinal motor neurons were isolated from rat embryos and transduced with lentiviruses encoding a mCherry reporter (control) and either miR-218 (miR-218 OE) or a miR-218 inhibitor (miR-218 KD). After 14 days *in vitro* confocal calcium imaging was performed to quantify frequency of spontaneous action potentials. (Figure 1A) Diagram of experimental design and (Figures 1B-E) time lapse images showing spontaneous activity measured with Fluo2 HighAff AM. (Figure 1F) Representative traces of individual motor neurons. (Figure 1G) Quantification of spontaneous firing events in individual motor neurons transduced with a control virus (61 neurons), miR-218 overexpression (79 neurons) or miR-218 knockdown virus (44 neurons). Error bars ± SEM; Two-sided student's t-test. *** *p*<*0.001;* **** *p*<*0.0001.*

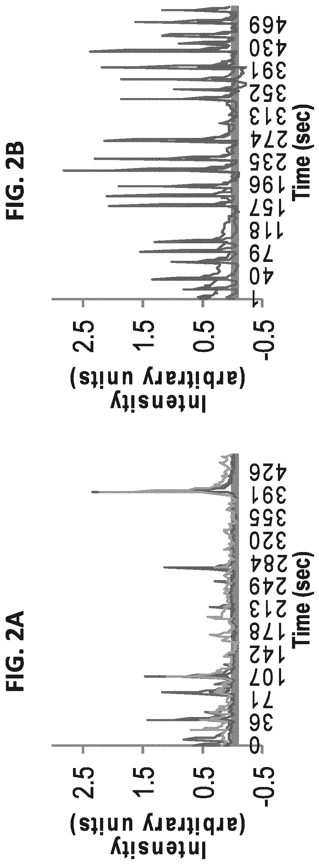

FIGs. 2A-B illustrate that miR-218 overexpression drives Hippocampal cells to a hyperexcitable state. Representative traces of individual Hippocampal neurons infected with control (Figure 2A) or miR-218 encoding (Figure 2B) lentiviruses. Hippocampal cells (isolated from P1 rat pups) were plated onto either poly-L-lysine or poly-omithine and laminin coated glass coverslips and grown in Neurobasal/B27 media supplemented with growth factors. At the day of plating, cells were infected with a control lentivirus (Figure 2A) or a lentivirus encoding miR-218 (Figure 2B) and a mCherry reporter. At 4 days *in vitro* (DIV) FUDR was added to inhibit the proliferation of glial cells. Media was changed on a weekly basis. At 14 DIV cultures were loaded with 2 µM Fluo2 HighAff AM, incubated for 1 hour at room temperature, washed in extracellular media and imaged using a Zeiss 710 confocal microscope. Fluo-2 calcium indicator (Teflabs) was added at a final concentration of 2 µM and cells were incubated for 45 minutes. Cells were washed twice and imaged at a wave length of 488 nm using a Zeiss 710 confocal microscope. Color variety distinguishes individual traces from different neurons in the same culture.

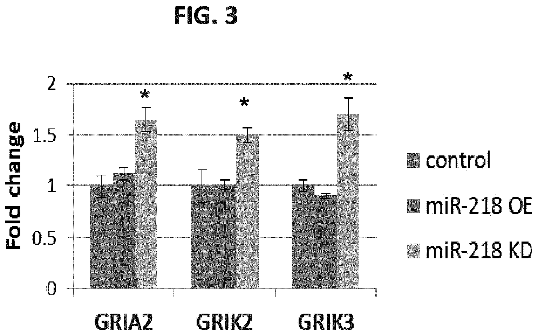

FIG. 3 is a bar graph illustrating a network of glutamate regulators downstream of miR-218. Primary motor neurons were infected with lentiviruses encoding for miR-218 (miR-218 OE), miR-218 inhibitor (miR-218 KD) or a control virus. 72 hours following the infection, RNA was extracted and subjected to qRT-PCR analysis. Error bars indicate standard deviation from 3 independent experiments in two technical duplicates. Asterisks indicate P < 0.05.

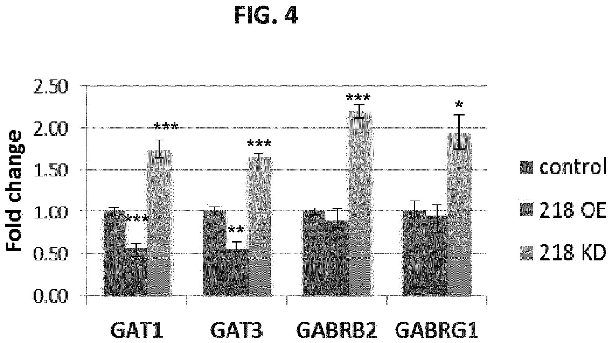

FIG. 4 is a bar graph illustrating a network of GABA (gamma-aminobutyric acid) regulators downstream of miR-218. Error bars indicate standard deviation from 3 independent experiments each with two technical duplicates. * p<0.05, ** p<0.005, *** p<0.0005.

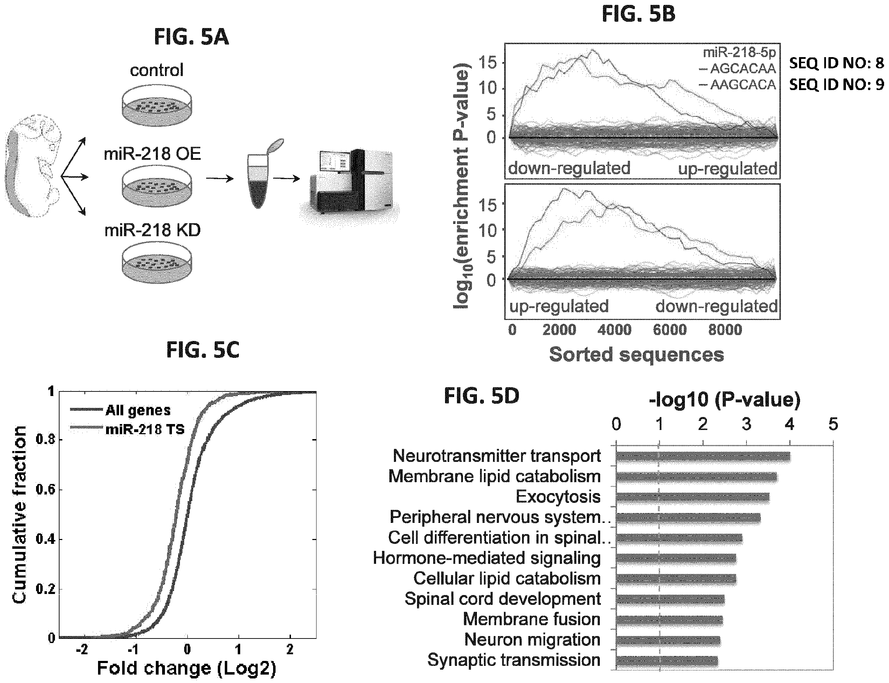

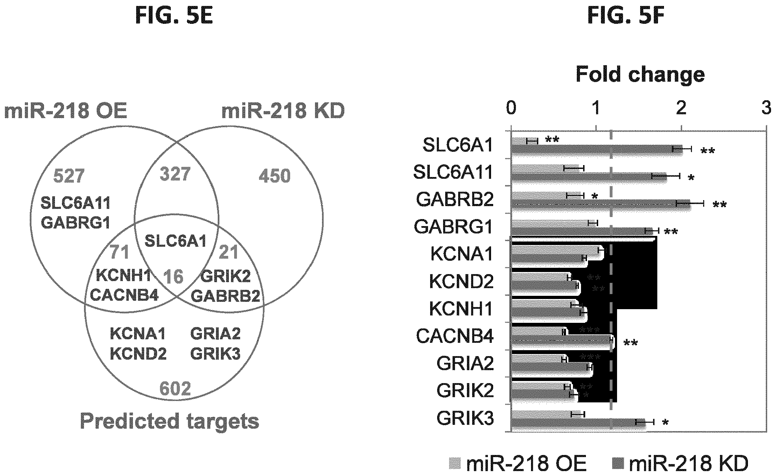

FIGs. 5A-F illustrate that miR-218 targets are predicted to regulate neuronal excitability. (Figure 5A) Diagram of experimental design for unbiased analysis of miR-218 targets in primary motor neurons. (Figure 5B) Sylamer analysis of enriched miRNA-recognition elements for all known miRNA in the 3'UTR of approximately 10,000 motor neuron mRNAs, ranked by differential expression upon miR-218 knockdown or miR-218 overexpression, relative to control virus. (Figure 5C) Plots of cumulative distribution function of miR-218 predicted targets (red) relative to all expressed mRNAs. Two-sided student's t-test *p*=4.12E⁻²⁸ (Figure 5D) miR-218 targets pathway enrichment analysis presented as -log10 (P-value). FAME algorithm. Dotted orange line indicates *P* = 0.05. (Figure 5E) Venn diagram of mRNAs down (/up) regulated following miR-218 overexpression (/knockdown) and predicted miR-218 targets. Genes related to neuronal excitability are marked in blue. (Figure 5F) qPCR analysis of miR-218 target mRNAs that are predicted to regulate neuronal excitability in primary motor neurons transduced with miR-218 (miR-218 OE), miR-218 knockdown (miR-218 KD) or a control lentivirus. Data normalized to average expression of HPRT and β-actin. Three independent experimental repeats in two technical duplicates. Error bar, ±SEM. Two-sided student's t-test. ^{∗}*p*<*0.05;* ^{∗∗}*p*<*0.01.*

### DESCRIPTION OF SPECIFIC EMBODIMENTS OF THE INVENTION

The present invention, in some embodiments thereof, relates to methods of treating motor neuron diseases and, more particularly, but not exclusively, to Amyotrophic Lateral Sclerosis (ALS).

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Motor neuron diseases (MND) belong to a group of neurological disorders attributed to the destruction of motor neurons of the central nervous system and degenerative changes in the motor neuron pathway. Micro-RNAs (also known as miRNAs) are 20- to 24-nucleotide (nt) RNA molecule members of the family of non-coding small RNAs. Micro-RNAs have been implicated in various neurological diseases as well as various cancers and precancerous conditions.

The present inventors have uncovered that miR-218 control motor neuron excitability (Figures 1A-G). Thus, overexpression of miR-218 derived primary motors to a hyperexcitable state, while knockdown of miR-218 silenced neuronal activity (Figure 1F-G). Previous reports showed that several miRNAs regulate neuronal excitability (e.g. miR-128, miR-223, miR-134 and miR-1000), however in all of these examples, miRNA overexpression reduced neuronal activity. In this respect miR-218 seems to be unique, as it is the only miRNA known to increase neuronal excitability. Moreover, next generation sequencing drove the identification of miR-218 target genes (Figures 3-4 and 5A-F), including KCND2, KCNH1, GABRB2, SLC6A1, SLC6A11, KCNA1, CACNB4, GRIA2, GRIK2, GABRG1 and GRIK3, thereby substantiating the use of miR-218 or modulating any of its target genes for therapy.

Thus, the present results shed light on the mechanisms by which miR-218 contributes to proper motor neuron function. Taken together, these observations establish a novel therapeutic role for miR-218 and its target genes (e.g. KCND2, KCNH1, GABRB2, SLC6A1, SLC6A11, KCNA1, CACNB4, GRIA2, GRIK2, GABRG1 and GRIK3) in the treatment of motor neuron diseases.

Thus, according to one aspect of the present invention, there is provided a use of an agent selected from the group consisting of miR-218 (also referred to herein as miR-218-5p), miR-218* (also referred to herein as miR-218-3p), precursor thereof and a polynucleotide sequence encoding miR-218 or miR-218* or precursor thereof, for the preparation of a medicament for the treatment of a motor neuron disease (MND).

According to an aspect of the invention there is provided a method of treating a subject having MND, the method comprising administering to the subject a therapeutically effective amount of an agent selected from the group consisting of miR-218, miR-218*, precursor thereof and a polynucleotide sequence encoding miR-218 or miR-218* or precursor thereof, thereby treating the MND in the subject.

According to an embodiment, the miR-218 is a micro-RNA molecule having a nucleic acid sequence as set forth in accession nos. MI0000294, MI0000295, MIMAT0000275, MIMAT0004565 or MIMAT0004566 (accession numbers at the miRbase). Exemplary miR-218 polynucleotide sequences are set forth in SEQ ID NOs: 1-5.

The term "microRNA", "miRNA", and "miR" are synonymous and refer to a collection of non-coding single-stranded RNA molecules of about 19-28 nucleotides in length, which regulate gene expression. miRNAs are found in a wide range of organisms (e.g. viruses, humans) and have been shown to play a role in development, homeostasis, and disease etiology.

Genes coding for miRNAs are transcribed leading to production of a miRNA precursor known as the pri-miRNA. The pri-miRNA is typically part of a polycistronic RNA comprising multiple pri-miRNAs. The pri-miRNA may form a hairpin with a stem and loop. The stem may comprise mismatched bases.

The hairpin structure of the pri-miRNA is recognized by Drosha, which is an RNase III endonuclease. Drosha typically recognizes terminal loops in the pri-miRNA and cleaves approximately two helical turns into the stem to produce a 60-70 nucleotide precursor known as the pre-miRNA. Drosha cleaves the pri-miRNA with a staggered cut typical of RNase III endonucleases yielding a pre-miRNA stem loop with a 5' phosphate and approximately 2 nucleotide 3' overhang. It is estimated that approximately one helical turn of stem (approximately 10 nucleotides) extending beyond the Drosha cleavage site is essential for efficient processing. The pre-miRNA is then actively transported from the nucleus to the cytoplasm by Ran-GTP and the export receptor Ex-portin-5.

The double-stranded stem of the pre-miRNA is then recognized by Dicer, which is also an RNase III endonuclease. Dicer may also recognize the 5' phosphate and 3' overhang at the base of the stem loop. Dicer then cleaves off the terminal loop two helical turns away from the base of the stem loop leaving an additional 5' phosphate and approximately 2 nucleotide 3' overhang. The resulting siRNA-like duplex, which may comprise mismatches, comprises the mature miRNA and a similar-sized fragment known as the miRNA*. The miRNA and miRNA* may be derived from opposing arms of the pri-miRNA and pre-miRNA. MiRNA* sequences may be found in libraries of cloned miRNAs but typically at lower frequency than the miRNAs.

Although initially present as a double-stranded species with miRNA*, the miRNA eventually become incorporated as a single-stranded RNA into a ribonucleoprotein complex known as the RNA-induced silencing complex (RISC). Various proteins can form the RISC, which can lead to variability in specificity for miRNA/miRNA* duplexes, binding site of the target gene, activity of miRNA (repress or activate), and which strand of the miRNA/miRNA* duplex is loaded in to the RISC.

When the miRNA strand of the miRNA:miRNA* duplex is loaded into the RISC, the miRNA* is removed and degraded. The strand of the miRNA:miRNA* duplex that is loaded into the RISC is the strand whose 5' end is less tightly paired. In cases where both ends of the miRNA:miRNA* have roughly equivalent 5' pairing, both miRNA and miRNA* may have gene silencing activity.

The RISC identifies target nucleic acids based on high levels of complementarity between the miRNA and the mRNA, especially by nucleotides 2-7 of the miRNA.

A number of studies have looked at the base-pairing requirement between miRNA and its mRNA target for achieving efficient inhibition of translation (reviewed by Bartel 2004, Cell 116-281). In mammalian cells, the first 8 nucleotides of the miRNA may be important (Doench & Sharp 2004 GenesDev 2004-504). However, other parts of the microRNA may also participate in mRNA binding. Moreover, sufficient base pairing at the 3' can compensate for insufficient pairing at the 5' (Brennecke et al, 2005 PLoS 3-e85). Computation studies, analyzing miRNA binding on whole genomes have suggested a specific role for bases 2-7 at the 5' of the miRNA in target binding but the role of the first nucleotide, found usually to be "A" was also recognized (Lewis et at 2005 Cell 120-15). Similarly, nucleotides 1-7 or 2-8 were used to identify and validate targets by Krek et al (2005, Nat Genet 37-495).

The target sites in the mRNA may be in the 5' UTR, the 3' UTR or in the coding region. Interestingly, multiple miRNAs may regulate the same mRNA target by recognizing the same or multiple sites. The presence of multiple miRNA binding sites in most genetically identified targets may indicate that the cooperative action of multiple RISCs provides the most efficient translational inhibition.

MiRNAs may direct the RISC to downregulate gene expression by either of two mechanisms: mRNA cleavage or translational repression. The miRNA may specify cleavage of the mRNA if the mRNA has a certain degree of complementarity to the miRNA. When a miRNA guides cleavage, the cut is typically between the nucleotides pairing to residues 10 and 11 of the miRNA. Alternatively, the miRNA may repress translation if the miRNA does not have the requisite degree of complementarity to the miRNA. Translational repression may be more prevalent in animals since animals may have a lower degree of complementarity between the miRNA and binding site.

Thus, naturally occurring pre-miRNAs are generated from longer primary transcripts (pri-miRNAs - accession number at the miRbase = MI0000294 or MI0000295) by a ribonuclease, e.g., Drosha.

It should be noted that there may be variability in the 5' and 3' ends of any pair of miRNA and miRNA*. This variability may be due to variability in the enzymatic processing of Drosha and Dicer with respect to the site of cleavage. Variability at the 5' and 3' ends of miRNA and miRNA* may also be due to mismatches in the stem structures of the pri-miRNA and pre-miRNA. The mismatches of the stem strands may lead to a population of different hairpin structures. Variability in the stem structures may also lead to variability in the products of cleavage by Drosha and Dicer.

The term "microRNA mimic" refers to synthetic non-coding RNAs that are capable of entering the RNAi pathway and regulating gene expression. miRNA mimics imitate the function of endogenous microRNAs (miRNAs) and can be designed as mature, double stranded molecules or mimic precursors (e.g., or pre-miRNAs). miRNA mimics can be comprised of modified or unmodified RNA, DNA, RNA-DNA hybrids, or alternative nucleic acid chemistries (e.g., LNAs or 2'-O,4'-C-ethylene-bridged nucleic acids (ENA)). For mature, double stranded miRNA mimics, the length of the duplex region can vary between 13-33, 18-24 or 21-23 nucleotides. The miRNA may also comprise a total of at least 5, 6, 7, 8, 9, 10, 11, 12, 13, 14, 15, 16, 17, 18, 19,20,21,22, 23, 24, 25, 26, 27, 28, 29, 30, 31, 32, 33, 34, 35, 36, 37, 38, 39 or 40 nucleotides. The sequence of the miRNA may be the first 13-33 nucleotides of the pre-miRNA. The sequence of the miRNA may also be the last 13-33 nucleotides of the pre-miRNA. The sequence of the miRNA may comprise the sequence of SEQ ID NOS: 1-5 or variants thereof.

It will be appreciated from the description provided herein above, that contacting cells (e.g. human cells) with a miRNA may be affected in a number of ways:
1. Transiently transfecting the cells (e.g. human cells) with the mature double stranded miRNA (e.g. SEQ ID NOs: 3-5);
2. Stably, or transiently transfecting the cells (e.g. human cells) with an expression vector which encodes the mature miRNA;
3. Stably, or transiently transfecting the cells (e.g. human cells) with an expression vector which encodes the pre-miRNA (e.g. SEQ ID NOs: 1-2) The pre-miRNA sequence may comprise from 45-90, 60-80 or 60-70 nucleotides. The sequence of the pre-miRNA may comprise a miRNA and a miRNA* as set forth herein. The sequence of the pre-miRNA may also be that of a pri-miRNA excluding from 0-160 nucleotides from the 5' and 3' ends of the pri-miRNA.
4. Stably, or transiently transfecting the cells (e.g. human cells) with an expression vector which encodes the pri-miRNA The pri-miRNA sequence may comprise from 45-30,000, 50-25,000, 100-20,000, 1,000-1,500 or 80-100 nucleotides. The sequence of the pri-miRNA may comprise a pre-miRNA, miRNA and miRNA*, as set forth herein, and variants thereof.

Preparation of miRNAs mimics can be effected by chemical synthesis methods or by recombinant methods.

It will be appreciated from the description provided herein above that contacting cells (e.g. human cells) with a miRNA may be effected by transfecting the cells with e.g. the mature double stranded miRNA, the pre-miRNA or the pri-miRNA.

The pre-miRNA sequence may comprise from 45-90, 60-80 or 60-70 nucleotides.

The pri-miRNA sequence may comprise from 45-30,000, 50-25,000, 100-20,000, 1,000-1,500 or 80-100 nucleotides.

According to a specific embodiment, miR-218 or miR-218* are used to downregulate the expression of a gene product (e.g. KCND2, KCNH1, GABRB2, SLC6A1, SLC6A11, KCNA1, CACNB4, GRIA2, GRIK2, GABRG1 or GRIK3).

According to another aspect of the present invention, there is provided a use of an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, for the preparation of a medicament for the treatment of MND.

According to another aspect of the present invention, there is provided a method of treating a subject having MND, the method comprising administering to the subject a therapeutically effective amount of an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, thereby treating the MND in the subject.

According to one embodiment, the gene product (i.e. target gene) comprises a human gene of a potassium channel, an ionotropic glutamate receptor subunit, a GABA receptor subunit, a GABA transporter and/or a calcium channel.

According to a specific embodiment, the human gene of the potassium channel comprises KCNA1 (Kv1.1), KCND2 (Kv4.2) or KCNH1 (Kv10.1).

According to a specific embodiment, the human gene of the ionotropic glutamate receptor subunit comprises GRIA2 (GluA2), GRIK2 (GluK2) or GRIK3 (GluK3).

According to a specific embodiment, the human gene of the GABA receptor subunit comprises GABRB2 or GABRG1.

According to a specific embodiment, the human gene of the GABA transporter comprises SLC6A1 (GAT1) or SLC6A11 (GAT3).

According to a specific embodiment, the human gene of the calcium channel comprises CACNB4.

As used herein the term "KCND2" refers to the human Potassium Channel, Voltage Gated Shal Related Subfamily D, Member 2, (also termed Kv4.2) having a nucleic acid sequence as set forth e.g. in accession nos: XM_011516166.1, NM_012281.2, XM_005250322.2, XM_011516167.1 or XM_011516165.1.

As used herein the term "KCNH1" refers to the human Potassium Channel, Voltage Gated Eag Related Subfamily H, Member 1, (also termed Kv10.1) having a nucleic acid sequence as set forth e.g. in accession nos: NM_172362.2, XM_011509514.1 or NM_002238.3.

As used herein the term "KCNA1" refers to the human Potassium Channel, Voltage Gated Shaker Related Subfamily A, (also termed Kv1.1) having a nucleic acid sequence as set forth e.g. in accession no: NM_000217.2.

As used herein the term "GABRB2" refers to the human Gamma-Aminobutyric Acid (GABA) A Receptor, Beta 2, having a nucleic acid sequence as set forth e.g. in accession nos: XM_011534501.1, NM_000813.2 or NM_021911.2.

As used herein the term "GABRG1" refers to the human Gamma-Aminobutyric Acid (GABA) A Receptor, Gamma 1, having a nucleic acid sequence as set forth e.g. in accession no: NM_173536.3.

As used herein the term "SLC6A1" refers to the human Solute Carrier Family 6 (Neurotransmitter Transporter), Member 1 (also termed GAT1), having a nucleic acid sequence as set forth e.g. in accession nos: NM_003042.3, XM_006713306.2, XM_011534027.1, XM_011534025.1, XM_005265410.3, XM_011534028.1, XM_005265411.3 or XM_011534026.1.

As used herein the term "SLC6A11" refers to the human Solute Carrier Family 6 (Neurotransmitter Transporter), Member 11, (also termed GAT3) having a nucleic acid sequence as set forth e.g. in accession nos: XM_011534033.1, NM_014229.1 or XM_011534032.1.

As used herein the term "CACNB4" refers to the human Calcium Channel, Voltage-Dependent, Beta 4 Subunit, having a nucleic acid sequence as set forth e.g. in accession nos: XM_011511799.1, NM_000726.3, NM_001005746.2, NM_001005747.2, XM_011511797.1, NM_001145798.1, XM_011511796.1, XM_011511800.1, XM_011511795.1, XM_005246838.2, XM_011511798.1 or XM_006712731.1.

As used herein the term "GRIA2" refers to the human Aliases for GRIA2 Gene Glutamate Receptor, Ionotropic, AMPA 2, (also termed GluA2) having a nucleic acid sequence as set forth e.g. in accession nos: NM_000826.3, NM_001083619.1, XM_011531892.1 or NM_001083620.1.

As used herein the term "GRIK2" refers to the human Glutamate Receptor, Ionotropic, Kainate 2, (also termed GluK2) having a nucleic acid sequence as set forth e.g. in accession nos: XM_011535779.1, NM_001166247.1, XM_011535777.1, XM_011535780.1, XM_005266945.2, NM_021956.4, XM_011535781.1, XM_011535778.1, NM_175768.3, XM_005266946.2.

As used herein the term "GRIK3" refers to the human Glutamate Receptor, Ionotropic, Kainate 3, (also termed GluK3) having a nucleic acid sequence as set forth e.g. in accession nos: XM_011541295.1, NM_000831.3 or XM_011541294.1.

As used herein the phrase "dowregulates expression" refers to dowregulating the expression of a protein (e.g. KCND2, KCNH1, GABRB2, SLC6A1, SLC6A11, KCNA1, CACNB4, GRIA2, GRIK2, GABRG1 or GRIK3) at the genomic (e.g. homologous recombination and site specific endonucleases) and/or the transcript level using a variety of molecules which interfere with transcription and/or translation (e.g., RNA silencing agents) or on the protein level (e.g., aptamers, small molecules and inhibitory peptides, antagonists, enzymes that cleave the polypeptide, antibodies and the like).

For the same culture conditions the expression is generally expressed in comparison to the expression in a cell of the same species (e.g. human) but not contacted with the agent or contacted with a vehicle control, also referred to as control.

Down regulation of expression may be either transient or permanent.

According to specific embodiments, down regulating expression refers to the absence of mRNA and/or protein, as detected by RT-PCR or Western blot, respectively.

According to other specific embodiments down regulating expression refers to a decrease in the level of mRNA and/or protein, as detected by RT-PCR or Western blot, respectively. The reduction may be by at least a 10 %, at least 20 %, at least 30 %, at least 40 %, at least 50 %, at least 60 %, at least 70 %, at least 80 %, at least 90 %, at least 95 % or at least 99 % reduction.

Non-limiting examples of agents capable of down regulating a gene product (e.g. KCND2, KCNH1, GABRB2, SLC6A1, SLC6A11, KCNA1, CACNB4, GRIA2, GRIK2, GABRG1 or GRIK3) expression (e.g. in human beings) are described in details hereinbelow.

### Down-regulation at the nucleic acid level

Down-regulation at the nucleic acid level is typically effected using a nucleic acid agent, having a nucleic acid backbone, DNA, RNA, mimetics thereof or a combination of same. The nucleic acid agent may be encoded from a DNA molecule or provided to the cell (e.g. human cell) *per se.*

Thus, downregulation of a gene product (e.g. KCND2, KCNH1, GABRB2, SLC6A1, SLC6A11, KCNA1, CACNB4, GRIA2, GRIK2, GABRG1 or GRIK3) can be achieved by RNA silencing. As used herein, the phrase "RNA silencing" refers to a group of regulatory mechanisms [e.g. RNA interference (RNAi), transcriptional gene silencing (TGS), post-transcriptional gene silencing (PTGS), quelling, co-suppression, and translational repression] mediated by RNA molecules which result in the inhibition or "silencing" of the expression of a corresponding protein-coding gene. RNA silencing has been observed in many types of organisms, including plants, animals, and fungi.

As used herein, the term "RNA silencing agent" refers to an RNA which is capable of specifically inhibiting or "silencing" the expression of a target gene. In certain embodiments, the RNA silencing agent is capable of preventing complete processing (e.g, the full translation and/or expression) of an mRNA molecule through a post-transcriptional silencing mechanism. RNA silencing agents include non-coding RNA molecules, for example RNA duplexes comprising paired strands, as well as precursor RNAs from which such small non-coding RNAs can be generated. Exemplary RNA silencing agents include dsRNAs such as siRNAs, miRNAs and shRNAs.

In one embodiment, the RNA silencing agent is capable of inducing RNA interference.

In another embodiment, the RNA silencing agent is capable of mediating translational repression.

According to an embodiment of the invention, the RNA silencing agent is specific to the target RNA (e.g., KCND2, KCNH1, GABRB2, SLC6A1, SLC6A11, KCNA1, CACNB4, GRIA2, GRIK2, GABRG1 or GRIK3) and does not cross inhibit or silence other targets or a splice variant which exhibits 99% or less global homology to the target gene, e.g., less than 98%, 97%, 96%, 95%, 94%, 93%, 92%, 91%, 90%, 89%, 88%, 87%, 86%, 85%, 84%, 83%, 82%, 81% global homology to the target gene; as determined by PCR, Western blot, Immunohistochemistry and/or flow cytometry.

RNA interference refers to the process of sequence-specific post-transcriptional gene silencing in animals mediated by short interfering RNAs (siRNAs).

Following is a detailed description on RNA silencing agents that can be used according to specific embodiments of the present invention.

*DsRNA, siRNA and shRNA* - The presence of long dsRNAs in cells (e.g. human cells) stimulates the activity of a ribonuclease III enzyme referred to as dicer. Dicer is involved in the processing of the dsRNA into short pieces of dsRNA known as short interfering RNAs (siRNAs). Short interfering RNAs derived from dicer activity are typically about 21 to about 23 nucleotides in length and comprise about 19 base pair duplexes. The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex.

Accordingly, some embodiments of the invention contemplate use of dsRNA to downregulate protein expression from mRNA.

According to one embodiment dsRNA longer than 30 bp are used. Various studies demonstrate that long dsRNAs can be used to silence gene expression without inducing the stress response or causing significant off-target effects - see for example [Strat et al., Nucleic Acids Research, 2006, Vol. 34, No. 13 3803-3810; Bhargava A et al. Brain Res. Protoc. 2004;13:115-125; Diallo M., et al., Oligonucleotides. 2003;13:381-392; Paddison P.J., et al., Proc. Natl Acad. Sci. USA. 2002;99:1443-1448; Tran N., et al., FEBS Lett. 2004;573:127-134].

According to some embodiments of the invention, dsRNA is provided in cells where the interferon pathway is not activated, see for example Billy et al., PNAS 2001, Vol 98, pages 14428-14433. and Diallo et al, Oligonucleotides, October 1, 2003, 13(5): 381-392. doi:10.1089/154545703322617069.

According to an embodiment of the invention, the long dsRNA are specifically designed not to induce the interferon and PKR pathways for down-regulating gene expression. For example, Shinagwa and Ishii [Genes & Dev. 17 (11): 1340-1345, 2003] have developed a vector, named pDECAP, to express long double-strand RNA from an RNA polymerase II (Pol II) promoter. Because the transcripts from pDECAP lack both the 5'-cap structure and the 3'-poly(A) tail that facilitate ds-RNA export to the cytoplasm, long ds-RNA from pDECAP does not induce the interferon response.

Another method of evading the interferon and PKR pathways in mammalian systems is by introduction of small inhibitory RNAs (siRNAs) either via transfection or endogenous expression.

The term "siRNA" refers to small inhibitory RNA duplexes (generally between 18-30 base pairs) that induce the RNA interference (RNAi) pathway. Typically, siRNAs are chemically synthesized as 21mers with a central 19 bp duplex region and symmetric 2-base 3'-overhangs on the termini, although it has been recently described that chemically synthesized RNA duplexes of 25-30 base length can have as much as a 100-fold increase in potency compared with 21mers at the same location. The observed increased potency obtained using longer RNAs in triggering RNAi is suggested to result from providing Dicer with a substrate (27mer) instead of a product (21mer) and that this improves the rate or efficiency of entry of the siRNA duplex into RISC.

It has been found that position of the 3'-overhang influences potency of an siRNA and asymmetric duplexes having a 3'-overhang on the antisense strand are generally more potent than those with the 3'-overhang on the sense strand (Rose et al., 2005). This can be attributed to asymmetrical strand loading into RISC, as the opposite efficacy patterns are observed when targeting the antisense transcript.

The strands of a double-stranded interfering RNA (e.g., an siRNA) may be connected to form a hairpin or stem-loop structure (e.g., an shRNA). Thus, as mentioned, the RNA silencing agent of some embodiments of the invention may also be a short hairpin RNA (shRNA).

The term "shRNA", as used herein, refers to an RNA agent having a stem-loop structure, comprising a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The number of nucleotides in the loop is a number between and including 3 to 23, or 5 to 15, or 7 to 13, or 4 to 9, or 9 to 11. Some of the nucleotides in the loop can be involved in base-pair interactions with other nucleotides in the loop. Examples of oligonucleotide sequences that can be used to form the loop include 5'-CAAGAGA-3' and 5'-UUACAA-3' (International Patent Application Nos. WO2013126963 and WO2014107763). It will be recognized by one of skill in the art that the resulting single chain oligonucleotide forms a stem-loop or hairpin structure comprising a double-stranded region capable of interacting with the RNAi machinery.

Synthesis of RNA silencing agents suitable for use with some embodiments of the invention can be effected as follows. First, the mRNA sequence is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (www.ambion.com/techlib/tn/91/912.html).

Second, potential target sites are compared to an appropriate genomic database (e.g., human, mouse, rat etc.) using any sequence alignment software, such as the BLAST software available from the NCBI server (www.ncbi.nlm.nih.gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

For example, suitable siRNAs directed against e.g., KCND2, KCNH1, GABRB2, SLC6A1, SLC6A11, KCNA1, CACNB4, GRIA2, GRIK2, GABRG1 or GRIK3 can be commercially obtained from e.g. Santa Cruz Biotechnology (SCBT) or OriGene.

It will be appreciated that, and as mentioned hereinabove, the RNA silencing agent of some embodiments of the invention need not be limited to those molecules containing only RNA, but further encompasses chemically-modified nucleotides and non-nucleotides.
*miRNA and miRNA mimics* - According to another embodiment the RNA silencing agent may be a miRNA.

According to an embodiment, the miRNA is miR-218 or miR-218*, as mentioned above.

According to one embodiment, for regulation of SLC6A1, the miRNA is e.g. mir-4745, mir-1538, mir-4285, mir-151b, mir-151a, miR-323 or let-7f.

According to one embodiment, for regulation of SLC6A11, the miRNA is e.g. miR-15 or miR-579.

According to one embodiment, for regulation of GRIA2, the miRNA is e.g. mir-181b, mir-124, miR-4326 or miR-4312.

According to one embodiment, for regulation of GRIK2, the miRNA is e.g. mir-26b, miR-145* or miR-3120.

According to one embodiment, for regulation of GRIK3, the miRNA is e.g. miR-130b*, miR-185*, mir-1278, mir-1277, mir-190a, mir-1185-2 or mir-1185-1.

According to one embodiment, for regulation of KCNA1, the miRNA is e.g. miR-1193, miR-4324, mir-301b, mir-362, mir-7b, mir-15a, or let-7b.

According to one embodiment, for regulation of KCND2, the miRNA is e.g. miR-218 or miR-4289.

According to one embodiment, for regulation of KCNH1, the miRNA is e.g. miR-3154, miR-3143, mir-296, mir-335, mir-34a, mir-7704, or mir-615.

According to one embodiment, for regulation of CACNB4, the miRNA is e.g. miR-103a-2*, miR-218-1*, or mir-26b.

According to one embodiment, for regulation of GABRB2, the miRNA is e.g. miR-502, miR-554, mir-548s, mir-3926, mir-4698, mir-8063, or mir-5197.

According to one embodiment, for regulation of GABRG1, the miRNA is e.g. miR-3165, miR-505, mir-3924, mir-297, mir-190a, mir-1185-2, or mir-1185-1.

*Antisense* - Antisense is a single stranded RNA designed to prevent or inhibit expression of a gene by specifically hybridizing to its mRNA. Downregulation of e.g., KCND2, KCNH1, GABRB2, SLC6A1, SLC6A11, KCNA1, CACNB4, GRIA2, GRIK2, GABRG1 or GRIK3 can be effected using an antisense polynucleotide capable of specifically hybridizing with an mRNA transcript encoding e.g., KCND2, KCNH1, GABRB2, SLC6A1, SLC6A11, KCNA1, CACNB4, GRIA2, GRIK2, GABRG1 or GRIK3.

Design of antisense molecules which can be used to efficiently downregulate a gene e.g., KCND2, KCNH1, GABRB2, SLC6A1, SLC6A11, KCNA1, CACNB4, GRIA2, GRIK2, GABRG1 or GRIK3 must be effected while considering two aspects important to the antisense approach. The first aspect is delivery of the oligonucleotide into the cytoplasm of the appropriate cells (e.g. human cells), while the second aspect is design of an oligonucleotide which specifically binds the designated mRNA within cells (e.g. human cells) in a way which inhibits translation thereof.

The prior art teaches of a number of delivery strategies which can be used to efficiently deliver oligonucleotides into a wide variety of cell types [see, for example, Jääskeläinen et al. Cell Mol Biol Lett. (2002) 7(2):236-7; Gait, Cell Mol Life Sci. (2003) 60(5):844-53; Martino et al. J Biomed Biotechnol. (2009) 2009:410260; Grijalvo et al. Expert Opin Ther Pat. (2014) 24(7):801-19; Falzarano et al, Nucleic Acid Ther. (2014) 24(1):87-100; Shilakari et al. Biomed Res Int. (2014) 2014: 526391; Prakash et al. Nucleic Acids Res. (2014) 42(13):8796-807 and Asseline et al. J Gene Med. (2014) 16(7-8):157-65]

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)]. Such algorithms have been successfully used to implement an antisense approach in cells (e.g. human cells).

In addition, several approaches for designing and predicting efficiency of specific oligonucleotides using an in vitro system were also published (Matveeva et al., Nature Biotechnology 16: 1374 - 1375 (1998)].

Thus, the generation of highly accurate antisense design algorithms and a wide variety of oligonucleotide delivery systems, enable an ordinarily skilled artisan to design and implement antisense approaches suitable for downregulating expression of known sequences without having to resort to undue trial and error experimentation.

In some embodiments, the RNA silencing agent provided herein can be functionally associated with a cell-penetrating peptide." As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell (e.g. human cell). The cell-penetrating peptide used in the membrane-permeable complex of some embodiments of the invention preferably comprises at least one non-functional cysteine residue, which is either free or derivatized to form a disulfide link with a double-stranded ribonucleic acid that has been modified for such linkage. Representative amino acid motifs conferring such properties are listed in U.S. Pat. No. 6,348,185, the contents of which are expressly incorporated herein by reference. The cell-penetrating peptides of some embodiments of the invention preferably include, but are not limited to, penetratin, transportan, pIsl, TAT(48-60), pVEC, MTS, and MAP.

Nucleic acid agents can also operate at the DNA level as summarized infra.

Downregulation of a gene e.g., KCND2, KCNH1, GABRB2, SLC6A1, SLC6A11, KCNA1, CACNB4, GRIA2, GRIK2, GABRG1 or GRIK3 can also be achieved by inactivating the gene (e.g., KCND2, KCNH1, GABRB2, SLC6A1, SLC6A11, KCNA1, CACNB4, GRIA2, GRIK2, GABRG1 or GRIK3) via introducing targeted mutations involving loss-of function alterations (e.g. point mutations, deletions and insertions) in the gene structure.

As used herein, the phrase "loss-of-function alterations" refers to any mutation in the DNA sequence of a gene (e.g., KCND2, KCNH1, GABRB2, SLC6A1, SLC6A11, KCNA1, CACNB4, GRIA2, GRIK2, GABRG1 or GRIK3) which results in downregulation of the expression level and/or activity of the expressed product, i.e., the mRNA transcript and/or the translated protein. Non-limiting examples of such loss-of-function alterations include a missense mutation, *i.e*., a mutation which changes an amino acid residue in the protein with another amino acid residue and thereby abolishes the enzymatic activity of the protein; a nonsense mutation, *i.e*., a mutation which introduces a stop codon in a protein, e.g., an early stop codon which results in a shorter protein devoid of the enzymatic activity; a frame-shift mutation, *i.e.,* a mutation, usually, deletion or insertion of nucleic acid(s) which changes the reading frame of the protein, and may result in an early termination by introducing a stop codon into a reading frame (e.g., a truncated protein, devoid of the enzymatic activity), or in a longer amino acid sequence (e.g., a readthrough protein) which affects the secondary or tertiary structure of the protein and results in a non-functional protein, devoid of the enzymatic activity of the non-mutated polypeptide; a readthrough mutation due to a frame-shift mutation or a modified stop codon mutation (*i.e*., when the stop codon is mutated into an amino acid codon), with an abolished enzymatic activity; a promoter mutation, *i.e.,* a mutation in a promoter sequence, usually 5' to the transcription start site of a gene, which results in down-regulation of a specific gene product; a regulatory mutation, *i.e.,* a mutation in a region upstream or downstream, or within a gene, which affects the expression of the gene product; a deletion mutation, *i.e.,* a mutation which deletes coding nucleic acids in a gene sequence and which may result in a frame-shift mutation or an in-frame mutation (within the coding sequence, deletion of one or more amino acid codons); an insertion mutation, *i.e.,* a mutation which inserts coding or non-coding nucleic acids into a gene sequence, and which may result in a frame-shift mutation or an in-frame insertion of one or more amino acid codons; an inversion, *i.e.,* a mutation which results in an inverted coding or non-coding sequence; a splice mutation *i.e.,* a mutation which results in abnormal splicing or poor splicing; and a duplication mutation, *i.e.,* a mutation which results in a duplicated coding or non-coding sequence, which can be in-frame or can cause a frame-shift.

According to specific embodiments los-of-function alteration of a gene may comprise at least one allele of the gene.

The term "allele" as used herein, refers to any of one or more alternative forms of a gene locus, all of which alleles relate to a trait or characteristic. In a diploid cell or organism, the two alleles of a given gene occupy corresponding loci on a pair of homologous chromosomes.

According to other specific embodiments loss-of-function alteration of a gene comprises both alleles of the gene. In such instances the e.g., KCND2, KCNH1, GABRB2, SLC6A1, SLC6A11, KCNA1, CACNB4, GRIA2, GRIK2, GABRG1 or GRIK3 may be in a homozygous form or in a heterozygous form. According to this embodiment, homozygosity is a condition where both alleles at the e.g. KCND2, KCNH1, GABRB2, SLC6A1, SLC6A11, KCNA1, CACNB4, GRIA2, GRIK2, GABRG1 or GRIK3 locus are characterized by the same nucleotide sequence. Heterozygosity refers to different conditions of the gene at the e.g., KCND2, KCNH1, GABRB2, SLC6A1, SLC6A11, KCNA1, CACNB4, GRIA2, GRIK2, GABRG1 or GRIK3 locus.

Methods of introducing nucleic acid alterations to a gene of interest are well known in the art [see for example Menke D. Genesis (2013) 51: - 618; Capecchi, Science (1989) 244:1288-1292; Santiago et al. Proc Natl Acad Sci USA (2008) 105:5809-5814; International Patent Application Nos. WO 2014085593, WO 2009071334 and WO 2011146121; US Patent Nos. 8771945, 8586526, 6774279 and UP Patent Application Publication Nos. 20030232410, 20050026157, US20060014264; the contents of which are incorporated by reference in their entireties] and include targeted homologous recombination, site specific recombinases, PB transposases and genome editing by engineered nucleases. Agents for introducing nucleic acid alterations to a gene of interest can be designed publically available sources or obtained commercially from Transposagen, Addgene and Sangamo Biosciences.

Following is a description of various exemplary methods used to introduce nucleic acid alterations to a gene of interest and agents for implementing same that can be used according to specific embodiments of the present invention.

Genome Editing using engineered endonucleases - this approach refers to a reverse genetics method using artificially engineered nucleases to cut and create specific double-stranded breaks at a desired location(s) in the genome, which are then repaired by cellular endogenous processes such as, homology directed repair (HDS) and nonhomologous end-joining (NFfEJ). NFfEJ directly joins the DNA ends in a double-stranded break, while HDR utilizes a homologous sequence as a template for regenerating the missing DNA sequence at the break point. In order to introduce specific nucleotide modifications to the genomic DNA, a DNA repair template containing the desired sequence must be present during HDR. Genome editing cannot be performed using traditional restriction endonucleases since most restriction enzymes recognize a few base pairs on the DNA as their target and the probability is very high that the recognized base pair combination will be found in many locations across the genome resulting in multiple cuts not limited to a desired location. To overcome this challenge and create site-specific single- or double-stranded breaks, several distinct classes of nucleases have been discovered and bioengineered to date. These include the meganucleases, Zinc finger nucleases (ZFNs), transcription-activator like effector nucleases (TALENs) and CRISPR/Cas system.

*Meganucleases* - Meganucleases are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif. The four families of meganucleases are widely separated from one another with respect to conserved structural elements and, consequently, DNA recognition sequence specificity and catalytic activity. Meganucleases are found commonly in microbial species and have the unique property of having very long recognition sequences (>14bp) thus making them naturally very specific for cutting at a desired location. This can be exploited to make site-specific double-stranded breaks in genome editing. One of skill in the art can use these naturally occurring meganucleases, however the number of such naturally occurring meganucleases is limited. To overcome this challenge, mutagenesis and high throughput screening methods have been used to create meganuclease variants that recognize unique sequences. For example, various meganucleases have been fused to create hybrid enzymes that recognize a new sequence. Alternatively, DNA interacting amino acids of the meganuclease can be altered to design sequence specific meganucleases (see e.g., US Patent 8,021,867). Meganucleases can be designed using the methods described in e.g., Certo, MT et al. Nature Methods (2012) 9:073-975; U.S. Patent Nos. 8,304,222; 8,021,867; 8, 119,381; 8, 124,369; 8, 129,134; 8,133,697; 8,143,015; 8,143,016; 8, 148,098; or 8, 163,514, the contents of each are incorporated herein by reference in their entirety. Alternatively, meganucleases with site specific cutting characteristics can be obtained using commercially available technologies e.g., Precision Biosciences' Directed Nuclease Editor^{™} genome editing technology.

*ZFNs and TALENs* - Two distinct classes of engineered nucleases, zinc-finger nucleases (ZFNs) and transcription activator-like effector nucleases (TALENs), have both proven to be effective at producing targeted double-stranded breaks (Christian *et al.*, 2010; Kim *et al.*, 1996; Li *et al.*, 2011; Mahfouz *et al.*, 2011; Miller *et al.*, 2010).

Basically, ZFNs and TALENs restriction endonuclease technology utilizes a non-specific DNA cutting enzyme which is linked to a specific DNA binding domain (either a series of zinc finger domains or TALE repeats, respectively). Typically a restriction enzyme whose DNA recognition site and cleaving site are separate from each other is selected. The cleaving portion is separated and then linked to a DNA binding domain, thereby yielding an endonuclease with very high specificity for a desired sequence. An exemplary restriction enzyme with such properties is Fokl. Additionally Fokl has the advantage of requiring dimerization to have nuclease activity and this means the specificity increases dramatically as each nuclease partner recognizes a unique DNA sequence. To enhance this effect, Fokl nucleases have been engineered that can only function as heterodimers and have increased catalytic activity. The heterodimer functioning nucleases avoid the possibility of unwanted homodimer activity and thus increase specificity of the double-stranded break.

Thus, for example to target a specific site, ZFNs and TALENs are constructed as nuclease pairs, with each member of the pair designed to bind adjacent sequences at the targeted site. Upon transient expression in cells (e.g. human cells), the nucleases bind to their target sites and the Fokl domains heterodimerize to create a double-stranded break. Repair of these double-stranded breaks through the nonhomologous end-joining (NHEJ) pathway most often results in small deletions or small sequence insertions. Since each repair made by NHEJ is unique, the use of a single nuclease pair can produce an allelic series with a range of different deletions at the target site. The deletions typically range anywhere from a few base pairs to a few hundred base pairs in length, but larger deletions have successfully been generated in cell culture by using two pairs of nucleases simultaneously (Carlson *et al.*, 2012; Lee *et al.*, 2010). In addition, when a fragment of DNA with homology to the targeted region is introduced in conjunction with the nuclease pair, the double-stranded break can be repaired via homology directed repair to generate specific modifications (Li *et al.*, 2011; Miller *et al.*, 2010; Urnov *et al.*, 2005).

Although the nuclease portions of both ZFNs and TALENs have similar properties, the difference between these engineered nucleases is in their DNA recognition peptide. ZFNs rely on Cys2- His2 zinc fingers and TALENs on TALEs. Both of these DNA recognizing peptide domains have the characteristic that they are naturally found in combinations in their proteins. Cys2-His2 Zinc fingers typically found in repeats that are 3 bp apart and are found in diverse combinations in a variety of nucleic acid interacting proteins. TALEs on the other hand are found in repeats with a one-to-one recognition ratio between the amino acids and the recognized nucleotide pairs. Because both zinc fingers and TALEs happen in repeated patterns, different combinations can be tried to create a wide variety of sequence specificities. Approaches for making site-specific zinc finger endonucleases include, e.g., modular assembly (where Zinc fingers correlated with a triplet sequence are attached in a row to cover the required sequence), OPEN (low-stringency selection of peptide domains vs. triplet nucleotides followed by high-stringency selections of peptide combination vs. the final target in bacterial systems), and bacterial one-hybrid screening of zinc finger libraries, among others. ZFNs can also be designed and obtained commercially from e.g., Sangamo Biosciences^{™} (Richmond, CA).

Method for designing and obtaining TALENs are described in e.g. Reyon et al. Nature Biotechnology 2012 May;30(5):460-5; Miller et al. Nat Biotechnol. (2011) 29: 143-148; Cermak et al. Nucleic Acids Research (2011) 39 (12): e82 and Zhang et al. Nature Biotechnology (2011) 29 (2): 149-53. A recently developed web-based program named Mojo Hand was introduced by Mayo Clinic for designing TAL and TALEN constructs for genome editing applications (can be accessed through www.talendesign.org). TALEN can also be designed and obtained commercially from e.g., Sangamo Biosciences^{™} (Richmond, CA).

*CRISPR-Cas system* - Many bacteria and archea contain endogenous RNA-based adaptive immune systems that can degrade nucleic acids of invading phages and plasmids. These systems consist of clustered regularly interspaced short palindromic repeat (CRISPR) genes that produce RNA components and CRISPR associated (Cas) genes that encode protein components. The CRISPR RNAs (crRNAs) contain short stretches of homology to specific viruses and plasmids and act as guides to direct Cas nucleases to degrade the complementary nucleic acids of the corresponding pathogen. Studies of the type II CRISPR/Cas system of *Streptococcus pyogenes* have shown that three components form an RNA/protein complex and together are sufficient for sequence-specific nuclease activity: the Cas9 nuclease, a crRNA containing 20 base pairs of homology to the target sequence, and a trans-activating crRNA (tracrRNA) (Jinek et al. Science (2012) 337: 816-821.). It was further demonstrated that a synthetic chimeric guide RNA (gRNA) composed of a fusion between crRNA and tracrRNA could direct Cas9 to cleave DNA targets that are complementary to the crRNA in vitro. It was also demonstrated that transient expression of Cas9 in conjunction with synthetic gRNAs can be used to produce targeted double-stranded brakes in a variety of different species (Cho *et al.*, 2013; Cong *et al.*, 2013; DiCarlo *et al.*, 2013; Hwang *et al.*, 2013a,b; Jinek *et al.*, 2013; Mali *et al.*, 2013).

The CRIPSR/Cas system for genome editing contains two distinct components: a gRNA and an endonuclease e.g. Cas9.

The gRNA is typically a 20 nucleotide sequence encoding a combination of the target homologous sequence (crRNA) and the endogenous bacterial RNA that links the crRNA to the Cas9 nuclease (tracrRNA) in a single chimeric transcript. The gRNA/Cas9 complex is recruited to the target sequence by the base-pairing between the gRNA sequence and the complement genomic DNA. For successful binding of Cas9, the genomic target sequence must also contain the correct Protospacer Adjacent Motif (PAM) sequence immediately following the target sequence. The binding of the gRNA/Cas9 complex localizes the Cas9 to the genomic target sequence so that the Cas9 can cut both strands of the DNA causing a double-strand break. Just as with ZFNs and TALENs, the double-stranded brakes produced by CRISPR/Cas can undergo homologous recombination or NHEJ.

The Cas9 nuclease has two functional domains: RuvC and HNH, each cutting a different DNA strand. When both of these domains are active, the Cas9 causes double strand breaks in the genomic DNA.

A significant advantage of CRISPR/Cas is that the high efficiency of this system coupled with the ability to easily create synthetic gRNAs enables multiple genes to be targeted simultaneously. In addition, the majority of cells carrying the mutation present biallelic mutations in the targeted genes.

However, apparent flexibility in the base-pairing interactions between the gRNA sequence and the genomic DNA target sequence allows imperfect matches to the target sequence to be cut by Cas9.

Modified versions of the Cas9 enzyme containing a single inactive catalytic domain, either RuvC- or HNH-, are called 'nickases'. With only one active nuclease domain, the Cas9 nickase cuts only one strand of the target DNA, creating a single-strand break or 'nick'. A single-strand break, or nick, is normally quickly repaired through the HDR pathway, using the intact complementary DNA strand as the template. However, two proximal, opposite strand nicks introduced by a Cas9 nickase are treated as a double-strand break, in what is often referred to as a 'double nick' CRISPR system. A double-nick can be repaired by either NHEJ or HDR depending on the desired effect on the gene target. Thus, if specificity and reduced off-target effects are crucial, using the Cas9 nickase to create a double-nick by designing two gRNAs with target sequences in close proximity and on opposite strands of the genomic DNA would decrease off-target effect as either gRNA alone will result in nicks that will not change the genomic DNA.

Modified versions of the Cas9 enzyme containing two inactive catalytic domains (dead Cas9, or dCas9) have no nuclease activity while still able to bind to DNA based on gRNA specificity. The dCas9 can be utilized as a platform for DNA transcriptional regulators to activate or repress gene expression by fusing the inactive enzyme to known regulatory domains. For example, the binding of dCas9 alone to a target sequence in genomic DNA can interfere with gene transcription.

There are a number of publically available tools available to help choose and/or design target sequences as well as lists of bioinformatically determined unique gRNAs for different genes in different species such as the Feng Zhang lab's Target Finder, the Michael Boutros lab's Target Finder (E-CRISP), the RGEN Tools: Cas-OFFinder, the CasFinder: Flexible algorithm for identifying specific Cas9 targets in genomes and the CRISPR Optimal Target Finder.

In order to use the CRISPR system, both gRNA and Cas9 should be expressed in a target cell (e.g. human cell). The insertion vector can contain both cassettes on a single plasmid or the cassettes are expressed from two separate plasmids. CRISPR plasmids are commercially available such as the px330 plasmid from Addgene.

"Hit and run" or "in-out" - involves a two-step recombination procedure. In the first step, an insertion-type vector containing a dual positive/negative selectable marker cassette is used to introduce the desired sequence alteration. The insertion vector contains a single continuous region of homology to the targeted locus and is modified to carry the mutation of interest. This targeting construct is linearized with a restriction enzyme at a one site within the region of homology, electroporated into the cells (e.g. human cells), and positive selection is performed to isolate homologous recombinants. These homologous recombinants contain a local duplication that is separated by intervening vector sequence, including the selection cassette. In the second step, targeted clones are subjected to negative selection to identify cells (e.g. human cells) that have lost the selection cassette via intrachromosomal recombination between the duplicated sequences. The local recombination event removes the duplication and, depending on the site of recombination, the allele either retains the introduced mutation or reverts to wild type. The end result is the introduction of the desired modification without the retention of any exogenous sequences.

The "double-replacement" or "tag and exchange" strategy - involves a two-step selection procedure similar to the hit and run approach, but requires the use of two different targeting constructs. In the first step, a standard targeting vector with 3' and 5' homology arms is used to insert a dual positive/negative selectable cassette near the location where the mutation is to be introduced. After electroporation and positive selection, homologously targeted clones are identified. Next, a second targeting vector that contains a region of homology with the desired mutation is electroporated into targeted clones, and negative selection is applied to remove the selection cassette and introduce the mutation. The final allele contains the desired mutation while eliminating unwanted exogenous sequences.

Site-Specific Recombinases - The Cre recombinase derived from the P1 bacteriophage and Flp recombinase derived from the yeast *Saccharomyces cerevisiae are* site-specific DNA recombinases each recognizing a unique 34 base pair DNA sequence (termed "Lox" and "FRT", respectively) and sequences that are flanked with either Lox sites or FRT sites can be readily removed via site-specific recombination upon expression of Cre or Flp recombinase, respectively. For example, the Lox sequence is composed of an asymmetric eight base pair spacer region flanked by 13 base pair inverted repeats. Cre recombines the 34 base pair lox DNA sequence by binding to the 13 base pair inverted repeats and catalyzing strand cleavage and religation within the spacer region. The staggered DNA cuts made by Cre in the spacer region are separated by 6 base pairs to give an overlap region that acts as a homology sensor to ensure that only recombination sites having the same overlap region recombine.

Basically, the site specific recombinase system offers means for the removal of selection cassettes after homologous recombination. This system also allows for the generation of conditional altered alleles that can be inactivated or activated in a temporal or tissue-specific manner. Of note, the Cre and Flp recombinases leave behind a Lox or FRT "scar" of 34 base pairs. The Lox or FRT sites that remain are typically left behind in an intron or 3' UTR of the modified locus, and current evidence suggests that these sites usually do not interfere significantly with gene function.

Thus, Cre/Lox and Flp/FRT recombination involves introduction of a targeting vector with 3' and 5' homology arms containing the mutation of interest, two Lox or FRT sequences and typically a selectable cassette placed between the two Lox or FRT sequences. Positive selection is applied and homologous recombinants that contain targeted mutation are identified. Transient expression of Cre or Flp in conjunction with negative selection results in the excision of the selection cassette and selects for cells (e.g. human cells) where the cassette has been lost. The final targeted allele contains the Lox or FRT scar of exogenous sequences.

Transposases - As used herein, the term "transposase" refers to an enzyme that binds to the ends of a transposon and catalyzes the movement of the transposon to another part of the genome.

As used herein the term "transposon" refers to a mobile genetic element comprising a nucleotide sequence which can move around to different positions within the genome of a single cell (e.g. human cell). In the process the transposon can cause mutations and/or change the amount of a DNA in the genome of the cell (e.g. human cell).

A number of transposon systems that are able to also transpose in cells e.g. vertebrates have been isolated or designed, such as Sleeping Beauty [Izsvak and Ivics Molecular Therapy (2004) 9, 147-156], piggyBac [Wilson et al. Molecular Therapy (2007) 15, 139-145], Tol2 [Kawakami et al. PNAS (2000) 97 (21): 11403-11408] or Frog Prince [Miskey et al. Nucleic Acids Res. Dec 1, (2003) 31(23): 6873-6881]. Generally, DNA transposons translocate from one DNA site to another in a simple, cut-and-paste manner. Each of these elements has their own advantages, for example, Sleeping Beauty is particularly useful in region-specific mutagenesis, whereas Tol2 has the highest tendency to integrate into expressed genes. Hyperactive systems are available for Sleeping Beauty and piggyBac. Most importantly, these transposons have distinct target site preferences, and can therefore introduce sequence alterations in overlapping, but distinct sets of genes. Therefore, to achieve the best possible coverage of genes, the use of more than one element is particularly preferred. The basic mechanism is shared between the different transposases, therefore we will describe piggyBac (PB) as an example.

PB is a 2.5 kb insect transposon originally isolated from the cabbage looper moth, *Trichoplusia ni.* The PB transposon consists of asymmetric terminal repeat sequences that flank a transposase, PBase. PBase recognizes the terminal repeats and induces transposition via a "cut-and-paste" based mechanism, and preferentially transposes into the host genome at the tetranucleotide sequence TTAA. Upon insertion, the TTAA target site is duplicated such that the PB transposon is flanked by this tetranucleotide sequence. When mobilized, PB typically excises itself precisely to reestablish a single TTAA site, thereby restoring the host sequence to its pretransposon state. After excision, PB can transpose into a new location or be permanently lost from the genome.

Typically, the transposase system offers an alternative means for the removal of selection cassettes after homologous recombination quit similar to the use Cre/Lox or Flp/FRT. Thus, for example, the PB transposase system involves introduction of a targeting vector with 3' and 5' homology arms containing the mutation of interest, two PB terminal repeat sequences at the site of an endogenous TTAA sequence and a selection cassette placed between PB terminal repeat sequences. Positive selection is applied and homologous recombinants that contain targeted mutation are identified. Transient expression of PBase removes in conjunction with negative selection results in the excision of the selection cassette and selects for cells where the cassette has been lost. The final targeted allele contains the introduced mutation with no exogenous sequences.

For PB to be useful for the introduction of sequence alterations, there must be a native TTAA site in relatively close proximity to the location where a particular mutation is to be inserted.

Genome editing using recombinant adeno-associated virus (rAAV) platform - this genome-editing platform is based on rAAV vectors which enable insertion, deletion or substitution of DNA sequences in the genomes of live mammalian cells (e.g. human cells). The rAAV genome is a single-stranded deoxyribonucleic acid (ssDNA) molecule, either positive- or negative-sensed, which is about 4.7 kb long. These single-stranded DNA viral vectors have high transduction rates and have a unique property of stimulating endogenous homologous recombination in the absence of double-strand DNA breaks in the genome. One of skill in the art can design a rAAV vector to target a desired genomic locus and perform both gross and/or subtle endogenous gene alterations in a cell (e.g. human cell). rAAV genome editing has the advantage in that it targets a single allele and does not result in any off-target genomic alterations. rAAV genome editing technology is commercially available, for example, the rAAV GENESIS^{™} system from Horizon^{™} (Cambridge, UK).

It will be appreciated that the agent can be a mutagen that causes random mutations and the cells (e.g. human cells) exhibiting downregulation of the expression level and/or activity of e.g., KCND2, KCNH1, GABRB2, SLC6A1, SLC6A11, KCNA1, CACNB4, GRIA2, GRIK2, GABRG1 or GRIK3 may be selected.

The mutagens may be, but are not limited to, genetic, chemical or radiation agents. For example, the mutagen may be ionizing radiation, such as, but not limited to, ultraviolet light, gamma rays or alpha particles. Other mutagens may include, but not be limited to, base analogs, which can cause copying errors; deaminating agents, such as nitrous acid; intercalating agents, such as ethidium bromide; alkylating agents, such as bromouracil; transposons; natural and synthetic alkaloids; bromine and derivatives thereof; sodium azide; psoralen (for example, combined with ultraviolet radiation). The mutagen may be a chemical mutagen such as, but not limited to, ICR191, 1,2,7,8-diepoxy-octane (DEO), 5-azaC, N-methyl-N-nitrosoguanidine (MNNG) or ethyl methane sulfonate (EMS).

Methods for qualifying efficacy and detecting sequence alteration are well known in the art and include, but not limited to, DNA sequencing, electrophoresis, an enzyme-based mismatch detection assay and a hybridization assay such as PCR, RT-PCR, RNase protection, in-situ hybridization, primer extension, Southern blot, Northern Blot and dot blot analysis.

Sequence alterations in a specific gene can also be determined at the protein level using e.g. chromatography, electrophoretic methods, immunodetection assays such as ELISA and western blot analysis and immunohistochemistry.

In addition, one ordinarily skilled in the art can readily design a knock-in/knockout construct including positive and/or negative selection markers for efficiently selecting transformed cells (e.g. human cells) that underwent a homologous recombination event with the construct. Positive selection provides a means to enrich the population of clones that have taken up foreign DNA. Non-limiting examples of such positive markers include glutamine synthetase, dihydrofolate reductase (DHFR), markers that confer antibiotic resistance, such as neomycin, hygromycin, puromycin, and blasticidin S resistance cassettes. Negative selection markers are necessary to select against random integrations and/or elimination of a marker sequence (e.g. positive marker). Non-limiting examples of such negative markers include the herpes simplex-thymidine kinase (HSV-TK) which converts ganciclovir (GCV) into a cytotoxic nucleoside analog, hypoxanthine phosphoribosyltransferase (HPRT) and adenine phosphoribosytransferase (ARPT).

### Down-regulation at the polypeptide level

According to specific embodiments the agent capable of downregulating a gene product (e.g., KCND2, KCNH1, GABRB2, SLC6A1, SLC6A11, KCNA1, CACNB4, GRIA2, GRIK2, GABRG1 or GRIK3) is an antibody or antibody fragment capable of specifically binding the gene product. Preferably, the antibody specifically binds at least one epitope of a gene product e.g., KCND2, KCNH1, GABRB2, SLC6A1, SLC6A11, KCNA1, CACNB4, GRIA2, GRIK2, GABRG1 or GRIK3. As used herein, the term "epitope" refers to any antigenic determinant on an antigen to which the paratope of an antibody binds. Epitopic determinants usually consist of chemically active surface groupings of molecules such as amino acids or carbohydrate side chains and usually have specific three dimensional structural characteristics, as well as specific charge characteristics.

The term "antibody" as used in this invention includes intact molecules as well as functional fragments thereof, such as Fab, F(ab')2, and Fv that are capable of binding to macrophages. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

As the gene product e.g., KCND2, KCNH1, GABRB2, SLC6A1, SLC6A11, KCNA1, CACNB4, GRIA2, GRIK2, GABRG1 or GRIK3 may be localized intracellularly, an antibody or antibody fragment capable of specifically binding the aforementioned gene product is typically an intracellular antibody.

Antibody fragments according to some embodiments of the invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

Another agent which can be used along with some embodiments of the invention to downregulate a gene product e.g., KCND2, KCNH1, GABRB2, SLC6A1, SLC6A11, KCNA1, CACNB4, GRIA2, GRIK2, GABRG1 or GRIK3 is an aptamer. As used herein, the term "aptamer" refers to double stranded or single stranded RNA molecule that binds to specific molecular target, such as a protein. Various methods are known in the art which can be used to design protein specific aptamers. The skilled artisan can employ SELEX (Systematic Evolution of Ligands by Exponential Enrichment) for efficient selection as described in Stoltenburg R, Reinemann C, and Strehlitz B (Biomolecular engineering (2007) 24(4):381-403).

Another agent capable of downregulating a gene product (e.g. KCND2, KCNH1, GABRB2, SLC6A1, SLC6A11, KCNA1, CACNB4, GRIA2, GRIK2, GABRG1 or GRIK3) would be any molecule which binds to and/or cleaves the protein (e.g. KCND2, KCNH1, GABRB2, SLC6A1, SLC6A11, KCNA1, CACNB4, GRIA2, GRIK2, GABRG1 or GRIK3). Such molecules can be a small molecule, an antagonist, or an inhibitory peptide.

As mentioned above, agents of the present invention which are capable of upregulating an activity or amount of miRNA-218 or miRNA-218* include, but are not limited to, modified or unmodified polynucleotides (including oligonucleotides of miR-218 or miR-218*, precursors thereof and polynucleotide sequences encoding same).

The term "polynucleotide" refers to a single-stranded or double-stranded oligomer or polymer of ribonucleic acid (RNA), deoxyribonucleic acid (DNA) or mimetics thereof. This term includes polynucleotides and/or oligonucleotides derived from naturally occurring nucleic acids molecules (e.g., RNA or DNA), synthetic polynucleotide and/or oligonucleotide molecules composed of naturally occurring bases, sugars, and covalent internucleoside linkages (e.g., backbone), as well as synthetic polynucleotides and/or oligonucleotides having non-naturally occurring portions, which function similarly to respective naturally occurring portions.

The polynucleotides (including oligonucleotides) designed according to the teachings of the present invention can be generated according to any oligonucleotide synthesis method known in the art, including both enzymatic syntheses or solid-phase syntheses. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example: Sambrook, J. and Russell, D. W. (2001), "Molecular Cloning: A Laboratory Manual"; Ausubel, R. M. et al., eds. (1994, 1989), "Current Protocols in Molecular Biology," Volumes I-III, John Wiley & Sons, Baltimore, Maryland; Perbal, B. (1988), "A Practical Guide to Molecular Cloning," John Wiley & Sons, New York; and Gait, M. J., ed. (1984), "Oligonucleotide Synthesis"; utilizing solid-phase chemistry, e.g. cyanoethyl phosphoramidite followed by deprotection, desalting, and purification by, for example, an automated trityl-on method or HPLC.

The polynucleotide of the present invention may be modified using various methods known in the art. However, measures are taken to ensure that the miR function is maintained.

For example, the oligonucleotides or polynucleotides of the present invention may comprise heterocylic nucleosides consisting of purines and the pyrimidines bases, bonded in a 3'-to-5' phosphodiester linkage.

Preferably used oligonucleotides or polynucleotides are those modified either in backbone, internucleoside linkages, or bases, as is broadly described hereinunder.

Specific examples of preferred oligonucleotides or polynucleotides useful according to this aspect of the present invention include oligonucleotides or polynucleotides containing modified backbones or non-natural internucleoside linkages. Oligonucleotides or polynucleotides having modified backbones include those that retain a phosphorus atom in the backbone, as disclosed in U.S. Pat. Nos.: 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

Preferred modified oligonucleotide or polynucleotide backbones include, for example: phosphorothioates; chiral phosphorothioates; phosphorodithioates; phosphotriesters; aminoalkyl phosphotriesters; methyl and other alkyl phosphonates, including 3'-alkylene phosphonates and chiral phosphonates; phosphinates; phosphoramidates, including 3'-amino phosphoramidate and aminoalkylphosphoramidates; thionophosphoramidates; thionoalkylphosphonates; thionoalkylphosphotriesters; and boranophosphates having normal 3'-5' linkages, 2'-5' linked analogues of these, and those having inverted polarity wherein the adjacent pairs of nucleoside units are linked 3'-5' to 5'-3' or 2'-5' to 5'-2'. Various salts, mixed salts, and free acid forms of the above modifications can also be used.

Alternatively, modified oligonucleotide or polynucleotide backbones that do not include a phosphorus atom therein have backbones that are formed by short-chain alkyl or cycloalkyl internucleoside linkages, mixed heteroatom and alkyl or cycloalkyl internucleoside linkages, or one or more short-chain heteroatomic or heterocyclic internucleoside linkages. These include those having morpholino linkages (formed in part from the sugar portion of a nucleoside); siloxane backbones; sulfide, sulfoxide, and sulfone backbones; formacetyl and thioformacetyl backbones; methylene formacetyl and thioformacetyl backbones; alkene-containing backbones; sulfamate backbones; methyleneimino and methylenehydrazino backbones; sulfonate and sulfonamide backbones; amide backbones; and others having mixed N, O, S and CH₂ component parts, as disclosed in U.S. Pat. Nos.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

Other oligonucleotides or polynucleotides which may be used according to the present invention are those modified in both sugar and the internucleoside linkage, *i.e.,* the backbone of the nucleotide units is replaced with novel groups. The base units are maintained for complementation with the appropriate polynucleotide target. An example of such an oligonucleotide mimetic includes a peptide nucleic acid (PNA). A PNA oligonucleotide refers to an oligonucleotide where the sugar-backbone is replaced with an amide-containing backbone, in particular an aminoethylglycine backbone. The bases are retained and are bound directly or indirectly to aza-nitrogen atoms of the amide portion of the backbone. United States patents that teach the preparation of PNA compounds include, but are not limited to, U.S. Pat. Nos. 5,539,082; 5,714,331; and 5,719,262; each of which is herein incorporated by reference. Other backbone modifications which may be used in the present invention are disclosed in U.S. Pat. No. 6,303,374.

Oligonucleotides or polynucleotides of the present invention may also include base modifications or substitutions. As used herein, "unmodified" or "natural" bases include the purine bases adenine (A) and guanine (G) and the pyrimidine bases thymine (T), cytosine (C), and uracil (U). "Modified" bases include but are not limited to other synthetic and natural bases, such as: 5-methylcytosine (5-me-C); 5-hydroxymethyl cytosine; xanthine; hypoxanthine; 2-aminoadenine; 6-methyl and other alkyl derivatives of adenine and guanine; 2-propyl and other alkyl derivatives of adenine and guanine; 2-thiouracil, 2-thiothymine, and 2-thiocytosine; 5-halouracil and cytosine; 5-propynyl uracil and cytosine; 6-azo uracil, cytosine, and thymine; 5-uracil (pseudouracil); 4-thiouracil; 8-halo, 8-amino, 8-thiol, 8-thioalkyl, 8-hydroxyl, and other 8-substituted adenines and guanines; 5-halo, particularly 5-bromo, 5-trifluoromethyl, and other 5-substituted uracils and cytosines; 7-methylguanine and 7-methyladenine; 8-azaguanine and 8-azaadenine; 7-deazaguanine and 7-deazaadenine; and 3-deazaguanine and 3-deazaadenine. Additional modified bases include those disclosed in: U.S. Pat. No. 3,687,808; Kroschwitz, J. I., ed. (1990),"The Concise Encyclopedia Of Polymer Science And Engineering," pages 858-859, John Wiley & Sons; Englisch et al. (1991), "Angewandte Chemie," International Edition, 30, 613; and Sanghvi, Y. S., "Antisense Research and Applications," Chapter 15, pages 289-302, S. T. Crooke and B. Lebleu, eds., CRC Press, 1993. Such modified bases are particularly useful for increasing the binding affinity of the oligomeric compounds of the invention. These include 5-substituted pyrimidines, 6-azapyrimidines, and N-2, N-6, and O-6-substituted purines, including 2-aminopropyladenine, 5-propynyluracil, and 5-propynylcytosine. 5-methylcytosine substitutions have been shown to increase nucleic acid duplex stability by 0.6-1.2 °C (Sanghvi, Y. S. et al. (1993), "Antisense Research and Applications," pages 276-278, CRC Press, Boca Raton), and are presently preferred base substitutions, even more particularly when combined with 2'-O-methoxyethyl sugar modifications.

According to a specific embodiment the polynucleotide of miR-218 or 218* comprises a modification selected from the group consisting of a modified sugar-phosphate backbone and a modified base.

According to some embodiments of the invention, the miR-218 or 218* comprises a modification in both a sugar and an internucleoside linkage.

According to some embodiments of the invention, the modification is selected from the group consisting of a phosphorothioate, a chiral phosphorothioate, a phosphorodithioate, a phosphotriester, an aminoalkyl phosphotriester, a methyl phosphonate, an alkyl phosphonate, a chiral phosphonate, a phosphinate, a phosphoramidate, an aminoalkylphosphoramidate, a thionophosphoramidate, a thionoalkylphosphonate, a thionoalkylphosphotriester, a boranophosphate, a phosphodiester, a 2'-O-methoxyethyl, a 2'-O-methyl, a 2'-fluoro, a locked nucleic acid (LNA), a peptide nucleic acid (PNA) and a 2'-Fluoroarabinooligonucleotides (FANA).

It will be appreciated that an RNA molecule can be also generated using recombinant techniques.

To express an exogenous polynucleotide (*i.e.,* to produce an RNA molecule), a nucleic acid sequence encoding the polynucleotide of the present invention is preferably ligated into a nucleic acid construct. Such a nucleic acid construct includes a promoter sequence for directing transcription of the polynucleotide sequence in the cell in a constitutive or inducible manner.

Constitutive promoters suitable for use with the present invention are promoter sequences which are active under most environmental conditions and most types of cells such as the cytomegalovirus (CMV) and Rous sarcoma virus (RSV). Inducible promoters suitable for use with the present invention include for example the tetracycline-inducible promoter (Zabala M, et al., Cancer Res. 2004, 64(8): 2799-804).

The nucleic acid construct (also referred to herein as an "expression vector") of the present invention includes additional sequences which render this vector suitable for replication and integration in prokaryotes, eukaryotes, or preferably both (e.g., shuttle vectors). In addition, typical cloning vectors may also contain a transcription and translation initiation sequence, transcription and translation terminator and a polyadenylation signal.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Preferably, the promoter utilized by the nucleic acid construct of the present invention is active in the specific cell population transformed. Examples of cell type-specific and/or tissue-specific promoters include promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166).

Various methods can be used to introduce the expression vector of the present invention into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Introduction of nucleic acids by viral infection offers several advantages over other methods such as lipofection and electroporation, since higher transfection efficiency can be obtained due to the infectious nature of viruses.

As mentioned above, the miRNAs of the invention may be used for the treatment of a motor neuron disease (MND).

As used herein, the terms "treating" or "treatment" include abrogating, substantially inhibiting, slowing or reversing the progression of a condition, substantially ameliorating clinical or aesthetical symptoms of a condition or substantially preventing the appearance of clinical or aesthetical symptoms of a condition (e.g., MND).

The phrase "motor neuron disease (MND)" as used herein, refers to a neurological disorder that selectively destroys motor neurons. As such, diseases such as Huntington's chorea are not classified as MNDs.

According to one embodiment, the MND is a neurodegenerative disease.

Examples of motor neuron diseases include, but are not limited to Amyotrophic Lateral Sclerosis (ALS), also known as Lou Gehrig's Disease, primary lateral sclerosis, progressive muscular atrophy, pseudobulbar palsy, progressive bulbar palsy, lower motor neuron disease and spinal muscular atrophy 1 (SMA1, Werdnig-Hoffmann Disease), Spinal Muscular Atrophy Type 2 (SMA2) and Spinal Muscular Atrophy Type 3 (SMA3, Kugelberg-Welander Disease) and Charcot-Marie-Tooth Disorders.

Additional diseases affecting motor neurons including Kennedy disease, post-polio syndrome and distinguish these from neurodegeneration and also differentiate diseases where muscle damage or denervation occur without motor neuron loss (neuropathies and myopathies).

Additional diseases affecting motor neurons which may be treated by the present teachings include epilepsy and/or seizures. According to one embodiment, the seizures are epileptic seizures. According to another embodiment, the epilepsy or seizures are associated with brain injury, stroke, brain tumors, infections of the brain, birth defects or genetic mutations.

According to one embodiment, the motor neuron disease (MND) is Amyotrophic Lateral Sclerosis (ALS).

According to a specific embodiment, the ALS may be familial (inherited) or sporadic.

As used herein, the term "subject" refers to an animal, preferably a mammal, most preferably a human being of any gender or age (e.g., infant, child or adult) who has been diagnosed with MND or is predisposed to MND. The subject may show preliminary signs of a MND, such as muscle fatigue or have a moderate or full blown late stage disease. Alternatively, the subject may have a genetic predisposition to the disease.

MND diagnosis may be effected using gold-standard methods as well as by analyzing the levels of disease typical markers such as miRNA-218 or miRNA-218*.

Gold standard methods include those that make up the El Escorial criteria. Other diagnostic methods that can be used in conjunction with the method of the present invention are those that involve transcranial magnetic stimulation (TMS). This noninvasive procedure creates a magnetic pulse inside the brain that stimulates motor activity in a certain area of the body. Electrodes taped to different areas of the body pick up and record the electrical activity in the muscles.

Below is a list of such tests:
1. Electromyography (EMG) is used to diagnose muscle and nerve dysfunction and spinal cord disease. It is also used to measure the speed at which impulses travel along a particular nerve. EMG records the electrical activity from the brain and/or spinal cord to a peripheral nerve root (found in the arms and legs) that controls muscles during contraction and at rest. Very fine wire electrodes are inserted one at a time into a muscle to assess changes in electrical voltage that occur during movement and when the muscle is at rest. The electrodes are attached to a recording instrument. Testing usually lasts about an hour or more, depending on the number of muscles and nerves to be tested.
2. EMG is usually done in conjunction with a nerve conduction velocity study. This procedure also measures electrical energy to test the nerve's ability to send a signal. A technician tapes two sets of flat electrodes on the skin over the muscles. The first set of electrodes is used to send small pulses of electricity (similar to a jolt from static electricity) to stimulate the nerve that directs a particular muscle. The second set of electrodes transmits the responding electrical signal to a recording machine. The physician then reviews the response to verify any nerve damage or muscle disease.
2. Laboratory screening tests of blood, urine, or other substances can rule out muscle diseases and other disorders that may have symptoms similar to those of MND. For example, analysis of the fluid that surrounds the brain and spinal cord can detect a number of disorders, including PPS. Blood tests may be ordered to measure levels of the protein creatine kinase (which is needed for the chemical reactions that produce energy for muscle contractions); high levels may help diagnose muscle diseases such as muscular dystrophy.
3. Magnetic resonance imaging (MRI) uses computer-generated radio waves and a powerful magnetic field to produce detailed images of body structures including tissues, organs, bones, and nerves. These images can help diagnose brain and spinal cord tumors, eye disease, inflammation, infection, and vascular irregularities that may lead to stroke. MRI can also detect and monitor degenerative disorders such as multiple sclerosis and can document brain injury from trauma. MRI is often used to rule out diseases other than the MNDs that affect the head, neck, and spinal cord.
4. Muscle or nerve biopsy can help confirm nerve disease and nerve regeneration. A small sample of the muscle or nerve is removed under local anesthetic and studied under a microscope. The sample may be removed either surgically, through a slit made in the skin, or by needle biopsy, in which a thin hollow needle is inserted through the skin and into the muscle. A small piece of muscle remains in the hollow needle when it is removed from the body. Although this test can provide valuable information about the degree of damage, it is an invasive procedure that may itself cause neuropathic side effects. Many experts do not believe that a biopsy is always needed for diagnosis.

Once diagnosis is substantiated, the subject is administered with the miR-218 or miR-218*, precursor thereof or a polynucleotide sequence encoding miR-218 or miR-218* or precursor thereof. Additionally or alternatively, the subject may be administered with an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3.

According to some embodiment the administering is effected into the central nervous system (CNS) of the subject.

According to some embodiment the agent is formulated for administration into the CNS of a subject in need thereof.

The agents of the present invention including the miRNAs of some embodiments of the present invention (or the expression vectors encoding same) may be administered to the patient *per se* or as part of a pharmaceutical composition, where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the miRNAs accountable for the biological effect (e.g. an agent capable of downregulating an activity or expression of a target gene, as described herein, or a miR-218 or miR-218^{∗}, precursor thereof or a polynucleotide sequence encoding miR-218 or miR-218* or precursor thereof, as described herein).

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

The term "tissue" refers to part of an organism consisting of an aggregate of cells having a similar structure and/or a common function. Examples include, but are not limited to, brain tissue and muscle tissue.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (e.g. an agent capable of downregulating an activity or expression of a target gene, as described herein, or a miR-218 or miR-218*, precursor thereof or a polynucleotide sequence encoding miR-218 or miR-218* or precursor thereof, as described herein) effective to prevent, alleviate or ameliorate symptoms of a MND (e.g., ALS) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Animal models of amyotrophic lateral sclerosis (ALS) provide a unique opportunity to study this incurable and fatal human disease both clinically and pathologically. Nonetheless, postmortem ALS tissue remains the "gold standard" against which pathologic findings in animal models must be compared. Four natural disease models have been most extensively studied, including three mouse models: motor neuron degeneration (Mnd), progressive motor neuronopathy (pmn), wobbler, and one canine model: hereditary canine spinal muscular atrophy (HCSMA). The wobbler mouse has been the most extensively studied of these models with analyses of clinical, pathological (perikaryon, axon, muscle), and biochemical features. Experimentally induced ALS animal models have allowed controlled testing of various neurotoxic, viral and immune-mediated mechanisms. Molecular techniques have recently generated mouse models in which genes relevant to the human disease or motor neuron biology have been manipulated. Transgenic mouse overexpressing the mutated SOD1 gene of FALS patients, provide significant insights into mechanisms of motor neuron degeneration in this disease (reviewed by Pioro Clin Neurosci. 1995-1996;3(6):375-85).

Of specific relevance is a new transgenic mouse model with mutant *TDP-43* appears to be similar to human sporadic amyotrophic lateral sclerosis, opening up new opportunities for research on targeted therapies, described in Talan Neurology Today: 19 November 2009 - Volume 9 - Issue 22 - p 10-11.

According to one embodiment, the mouse model is the A315T stop mouse model (Wegorzewska, 2009 PNAS 106, p. 18809-14) from Jackson Laboratories Inc.).

Another exemplary mouse model are the transgenic SOD1 (superoxide dismutase 1) mice (described in the Examples section which follows) which express a G93A mutant form of human SOD1. SOD1 mice (TgN-SOD1-G93A-1Gur) exhibit a phenotype similar to amyotrophic lateral sclerosis (ALS) in humans (Gurney, 1994, Science 264 p. 1772-5).

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

According to another aspect of the invention, there is provided an article of manufacture comprising an agent selected from the group consisting of miR-218, miR-218*, precursor thereof and a polynucleotide sequence encoding miR-218 or miR-218* or precursor thereof, and an additional anti-MND agent, being packaged in a packaging material and identified in print, in or on the packaging material for use in the treatment of MND.

According to another aspect of the invention, there is provided an article of manufacture comprising an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, thereby treating the MND, and an additional anti-MND agent, being packaged in a packaging material and identified in print, in or on the packaging material for use in the treatment of MND.

According to one embodiment, the agents (e.g. an agent capable of downregulating an activity or expression of a target gene, as described herein, or a miR-218, miR-218*, precursor thereof or a polynucleotide sequence encoding miR-218 or miR-218* or precursor thereof, as described herein, and an additional anti-MND agent) are packed in a single container.

According to another embodiment, the agents (e.g. an agent capable of downregulating an activity or expression of a target gene, as described herein, or a miR-218, miR-218*, precursor thereof and a polynucleotide sequence encoding miR-218 or miR-218* or precursor thereof, as described herein, and an additional anti-MND agent) are packed in a separate containers.

According to one embodiment, the additional anti-MND agent is an anti-ALS agent.

An exemplary anti-MND agent includes, but is not limited to, Riluzole (e.g. Rilutek^{®} or APO-Riluzole).

It will be appreciated that the miRNAs of the invention may be administered alone or in conjunction with other known treatment methods. Thus, for example, the miRNAs of this aspect of the present invention may be administered together with any anti-MND agent (e.g. anti-ALS agent) capable of alleviating, treating or slowing ALS disease progression.

An exemplary anti-ALS agent which may be used in accordance with the present teachings is Riluzole (e.g. Rilutek^{®}).

According to a specific embodiment, the method comprises administering to the subject a miR-218, miR-218*, precursor thereof or a polynucleotide sequence encoding miR-218 or miR-218* or precursor thereof, and an anti-ALS agent (e.g. Riluzole).

According to a specific embodiment, the method comprises administering to the subject an agent capable of downregulating an activity or expression of a gene product selected from the group consisting of KCND2, KCNH1, GABRB2, SLC6A1, SLC6A11, KCNA1, CACNB4, GRIA2, GRIK2, GABRG1 and GRIK3, and an anti-ALS agent (e.g. Riluzole).

It will be appreciated that when administering more than one agent to the subject, the agents may be administered concomitantly or at separate times (e.g. within minutes, hours, days, weeks or months of each other).

According to another embodiment, an agent which upregulates an activity or expression of miR-218 or miR-218* may be used.

To be considered a therapeutic agent, the candidate agents of the present invention typically up-regulate an activity or expression of miR-218 or miR-218* by at least 1.5 fold and more preferably by at least 2 fold.

Following selection of a candidate agent as a therapeutic agent for the treatment of an MND, the agent may be tested - for example on an animal model for the disease (as discussed above) and ultimately the agent may be tested in humans. Validation of therapeutic efficacy may then lead to the preparation of the candidate agent as a pharmaceutical composition.

In some embodiments, the agents disclosed herein are formulated to penetrate the blood brain barrier to reach the CNS.

In various embodiments the agents disclosed herein circumvent the blood-brain barrier (BBB) and are delivered directly to the CNS. In some embodiments the pharmaceutical composition disclosed herein is useful for delivery of the agent directly into the CNS by transport along a neural pathway to the CNS, or by way of a perivascular channel, a prelymphatic channel, or a lymphatic channel associated with the brain and/or spinal cord. In some embodiments the pharmaceutical composition disclosed herein delivers the agent to the cerebrospinal fluid and then subsequently to the CNS, including the brain, retina, optic nerve and/or spinal cord.

As used herein the term "about" refers to ± 10 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, though some sequences are expressed in a RNA sequence format (e.g., reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-Ill Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### GENERAL MATERIALS AND EXPERIMENTAL PROCEDURES

***Animals.*** C57BL/6 mice and Sprague-Dawley rats were maintained on a 12 hour light/dark cycle. All experiments were performed according to Weizmann Institute of Science guidelines and IACUC approval (protocol No. 03040512-1).

***Tissue processing.*** Mice were deeply anesthetized with ketamine/xylazine (0.25 mL, 10 % (vol/vol), administered i.p.) before organ perfusion with PBS and 2.5 % paraformaldehyde [PFA as previously described in Gage G.J. et al., J Vis Exp (2012) 65]. Spinal cords were removed along with the vertebrae and placed in 2.5 % PFA at 4 °C for 48 hours. The spinal cords were then dissected out of the vertebrae, cut at L4-L5 level and placed in 1.25 % PFA for an additional 24 hours at 4 °C. Spinal cords were then processed into paraffin blocks and sectioned on a microtome into a series of 7 µm sections.

***Immunostaining.*** Tissue sections were rehydrated in a series of decreasing ethanol concentrations, followed by water. For immunohistochemical staining of ChAT (Choline Acetyltransferase), after rehydration slides were blocked with Cas-Block (Thermo Fisher) according to the manufacturer's instructions and incubated overnight with goat anti-ChAT antibody (1:100, Chemicon). For signal amplification, biotin-conjugated donkey anti-goat (1:200, Jackson Immunoresearch) and Streptavidin-HRP (1:200, Perkin-Elmar) antibodies were used. For detection, DAB kit (Invitrogen) was used according to the manufacturer's instructions. Slides were mounted with Immu-mount (Shandon) and covered with a glass coverslip.
***miRNA in situ-hybridization.** In* situ-hybridization was performed as previously described in Pena, J.T., et al., Nat Methods (2009) 6(2): 139-41. Briefly, spinal cord sections were rehydrated in a series of decreasing ethanol concentrations, fixed in 4 % paraformaldehyde and treated with 2 µg/ml Proteinase K. Slides were further fixed in EDC (Sigma), acetylated in acetic anhydride/ triethanolamine solution and hybridized according to the manufacturer instructions in a hybridization buffer containing 40 nM of 5' and 3' DIG-labeled miR-218 LNA probe (Exiqon). Slides were then washed and incubated overnight at 4 °C with alkaline phosphatase-conjugated goat-anti-DIG Fab fragments (1:1000, Roche). Detection of *in situ* signal was performed with BCIP/NBT, as previously described in Obemosterer et al., Nat Protoc (2007) 2(6): 1508-14. Slides were mounted with Immu-mount (Shandon) and covered with a glass coverslip.

***Primary neuronal cultures.*** Primary motor neurons were isolated and cultured as previously described in Milligan, C. and D. Gifondorwa, Methods Mol Biol (2011) 793: 77-85. Briefly, Sprague-Dawley timed-pregnant females were sacrificed at rat embryonic day 14.5 (E14.5), and spinal cords were dissected and dissociated enzymatically with papain (2 mg/ml, Sigma). Motor neurons were separated over a gradient of Optiprep (Sigma) and plated on 13 mm coverslips (200,000 cells/ coverslip, Thermo scientific), pre-coated with 3 µg/ml poly-omithine (Sigma) and 3 µg/ml laminin (Gibco). Motor neurons were cultured with Neurobasal medium (Gibco) supplemented with 2 % B27 (Gibco), 2 % horse serum (Sigma), X1 Glutamax (Gibco) and 1 ng/ml CNTF and GDNF (Peprotech). For deep sequencing analysis, primary motor neurons were isolated from time pregnant C57BL/6 mice at embryonic day 13.5 (E13.5).

Primary hippocampal neurons were prepared from postnatal day 1 Sprague-Dawley pups. Pups were killed by decapitation and hippocampi were dissected in a solution containing 20 mM HEPES and 1 M HBSS. Hippocampi were then dissociated enzymatically with papain (3 mg/ml, Sigma) and mechanically. Cells were plated on polyethyleneimine (PEI, 1:500, Sigma) pre-coated coverslips at 150,000 cells/coverslip in plating media comprising of Neurobasal medium (Gibco) supplemented with 2 % B27 (Gibco), 5 % FBS (Biological industries), X1 Glutamax (Gibco) and 0.5 % Penicillin/streptomycin (Biological industries). At 1 day *in vitro* (DIV) half of the media was replaced with growing media comprising of Neurobasal medium (Gibco) supplemented with 2 % B27 (Gibco), X1 Glutamax (Gibco) and 0.5 % Penicillin/streptomycin (Biological industries). Primary motor neurons and primary hippocampal neurons were maintained in 5 % CO₂ humidified 37 °C incubator. At 6 DIV 5'-fluoro-2'-deoxyuridine (Sigma) was added to inhibit proliferation of non-neuronal cells.
***miR-218 lentiviral constructs.*** To generate miR-218 encoding lentivirus, a 625 bp BamHI-EcoRI fragment containing the genomic miR-218 locus was digested from miR-vec-218 plasmid as previously described in Voorhoeve, P.M., et al., Cell (2006) 124(6): 1169-81 and inserted into the pUltra-Hot vector (Addgene plasmid # 24130) downstream of the human Ubiqiutic C promoter and mCherry-P2A. To generate miR-218 inhibitor encoding lentivirus, an approximately 250 bp fragment containing miR-218 inhibitor downstream to a U6 promoter was PCR amplified from the miR-Zip-218 plasmid (SBI) using the following primers: Fw 5'-CGTACGTAAAGATGGCTGTGAGGGACAG-3' (SEQ ID NO: 6) and Rev 5'-CGTACGTAAGAGAGACCCAGTAGAAGCAAAAAG-3' (SEQ ID NO: 7), and subcloned into pGEM vector (Promega), and then cloned into the pUltra-Hot vector using the SnaBI restriction site in the 3'LTR.

***Lentiviral vector production and cell transduction.*** HEK-293T cells were transfected by calcium phosphate with the lentivairal vectors and packaging plasmids psPAX2 and pMD2.G (Addgene plasmid numbers 12259 and 12260). Supernatants containing viral particles were concentrated by ultracentrifugation, resuspended in DMEM media (Gibco) and titrated. Primary neuronal cultures were infected at a multiplicity of infection (MOI) of 1, one hour after plating.
***siRNA.*** siRNAs (Integrated DNA Technologies, Inc.) were encapsulated in Neuro9^{™} nanoparticles (Precision NanoSystems, Inc.) as previously described in Rungta, R.L., et al., Mol Ther Nucleic Acids (2013) 2: e136. Primary motor neurons were transfected with siRNA at a final concentration of 1 µg/ml, at 9 DIV.

***Calcium imaging.*** Primary motor neurons were imaged at 12 DIV. Primary hippocampal neurons were imaged at 15 DIV. Cells plated on coverslips were incubated in a solution containing: 129 mM NaCl, 4 mM KCl, 1 mM MgCl₂, 2 mM CaCl2, 4.2 mM glucose and 10 mM HEPES, with pH adjusted to 7.4 with NaOH, and osmolarity to 320 mOsm with sucrose. Fluo-2 calcium indicator (Teflabs) was added at a final concentration of 2 µM and cells were incubated for 45 minutes. Cells were washed twice and imaged at a wave length of 488 nm with a Zeiss 710 confocal laser scanning microscope, using a 40x objective.

***Automated immunocytofluorescence.*** Primary embryonic motor neurons were fixed and stained 72 hours post viral transduction. Cells were rinsed with phosphate buffered saline (PBS) 3 time using Biotek EL406 washer/dispenser for automated 96- / 384-well microplates, fixed with 4 % formaldehyde (ChemCruz^{™}) for 15 minutes, and then permeabilized with 0.1 % Triton X-100 (Sigma) in 2 % BSA blocking solution for 20 minutes, using a Bravo automated liquid handler (Agilent Technology). Following blocking, cells were incubated with anti-neuronal Class III beta Tubulin (Tuj 1) antibody (1:1000, MRB-435P, Covance) for 1.5 hours and then with anti Cy2-conjugated donkey anti-rabbit IgG (1:200, Jackson ImmunoResearch) secondary antibody for 1 hour. 4'6-Diamidino-2-phenylindole dihydrochloride (DAPI) (1 µg/ml, sigma) was added for 5 minutes before, following 3 cycles of automated PBS rinsing (BioTek). Two sites of each well were imaged using automated fluorescence microscope (ImageXpress Micro and MetaXpress software, Molecular Devices). MN counting was done using nuclear stain (DAPI) and identification of neurons positive to Tuj1 staining (Alexa488, FITC channel). Phenotypic parameters were quantified using Neurite Outgrowth and MWCS modules of MetaXpress High-Content Image Acquisition and Analysis Software (Molecular Devices) set to identify and measure only cellular processes connected to cell bodies. Statistical analysis was performed using Student's t-test.

***RNA analysis.*** Total RNA from cultured neurons was isolated using miRNeasy Mini Kit (Qiagen) and reverse transcribed using the miScript II RT Kit (Qiagen). Quantitative real-time PCR was performed with the LightCycler480 instrument (Roche), in more than 3 independent biological repeats and technical duplicates. miScript SYBR Green PCR (Qiagen) and KAPA SYBR fast qPCR (KAPA biosystems) kits were used for detection of miRNAs and mRNAs respectively. U6 and hypoxanthine phosphoribosyltransferase 1 (Hprt) were used as references for normalization of miRNA and mRNA levels, respectively. Statistical analysis was performed using Student's t-test. Primer sequences are described in Table 1, below.

**Table 1: Primers used for quantitative real-time PCR.**

| **Gene** | **Forward** | **Reverse** |
|---|---|---|
| miR-218-5p | TTGTGCTTGATCTAACCATGT (SEQ ID NO: 10) | NA |
| RNU6B (U6) | GATGACACGCAAATTCGTGAA (SEQ ID NO: 11) | NA |
| HPRT | CGAGATGCTATGAAGGAGATGG (SEQ ID NO: 12) | GTAATCCAGCAGGTCAGCAAAG (SEQ ID NO: 13) |
| GAPDH | CCTTTAGTGGGCCCTCGG (SEQ ID NO: 14) | GCCTGGAGAAACCTGCCAAG (SEQ ID NO: 15) |
| KCNA1 | TGAGCAGGAGGGGAATCAGA (SEQ ID NO: 16) | ACACCCTTACCAAGCGGATG (SEQ ID NO: 17) |
| KCND2 | CAAGTTCACCAGCATCCCT (SEQ ID NO: 18) | CCCGAAAATCTTCCCTGCTAT (SEQ ID NO: 19) |
| KCNH1 | ACCTGATTCTCACCTACAATCTG (SEQ ID NO: 20) | TCTTCCGTTTCATCCTCTCCT (SEQ ID NO: 21) |
| CACNB4 | CACCGTATCCCACAGCAAT (SEQ ID NO: 22) | GAGGTCATTAGGCTTCGTCTT (SEQ ID NO: 23) |
| SLC6A1 | CCCAGGGTGGCATTTATGTCT (SEQ ID NO: 24) | GGGTGTGAAAAAGGACCAGC (SEQ ID NO: 25) |
| SLC6A11 | GTGGGCATGAGTGGAACACA (SEQ ID NO: 26) | GATATAGCCAAGACCCGGCG (SEQ ID NO: 27) |
| GABRB2 | AAGATGCGCCTGGATGTCAA (SEQ ID NO: 28) | ATGCTGGAGGCATCATAGGC (SEQ ID NO: 29) |
| GABRG1 | GTCTTCCTTTTCTCTCCTTCCC (SEQ ID NO: 30) | TCATCTTCATCATCTGCTTTATCAATG (SEQ ID NO: 31) |
| GRIA2 | CTGACACCCCATATCGACAA (SEQ ID NO: 32) | TCCAAAAATTGCGTAGACTCCT (SEQ ID NO: 33) |
| GRIK2 | CATCAACAGAAACAGGACTTTGC (SEQ ID NO: 34) | GTGAAGGACCGAAGATAGCAG (SEQ ID NO: 35) |
| GRIK3 | GGATGAGACCTGTACTGTCAT (SEQ ID NO: 36) | ACCTTCTACGTGAACCTCTAC (SEQ ID NO: 37) |

***Deep sequencing analysis.*** cDNAs were sequenced on one lane of Illumina 2500 sequencing machine with 50 bp single read following Lavin, Y., et al., Cell (2014) 159(6): 1312-26. Fasta files for each sample were generated by the usage of Illumina CASAVA-1.8.2 software. Reads for each sample were mapped independently using TopHat2 version (v2.0.10) [as previously described in Kim, D., et al., Genome Biol (2013) 14(4): R36] against the mouse genome build mm9. Approximately, 85-90 % mapping rate was observed. Only uniquely mapped reads were used to determine the number of reads falling into each gene with the HTSeq-count script (0.6.1p1) [as previously described in Anders, S. et al., Bioinformatics (2015) 31(2): 166-9]. Differentially expressed genes were calculated with the DESeq2 package (v1.4.5) [as previously described in Love, M.I. et al. Genome Biol (2014) 15(12): 550]. Genes that were expressed at least on one sample were considered. Differentially expressed genes, were determined by P-adj < 0.05 and an absolute fold change > 1.5. Hierarchical clustering using Pearson dissimilarity and complete linkage was performed in order to explore a pattern of gene expression. Clustering analysis was performed with Matlab software (8.0.0.783).

### EXAMPLE 1

### miR-218 regulates motor neuron excitability

To explore the functions of miR-218 in primary motor neurons, live calcium-imaging experiments were performed. Primary motor neurons were isolated from E13.5 rat embryos (following the method described in Wiese S., et al., Nat Protoc (2010) 5(1): 31-8) and transduced with lentiviruses encoding miR-218 (miR-218-OE), a miR-218 inhibitor (miR-218-KD) or a control virus. At 12 days in culture, neuronal activity was analyzed by incubating neurons with Fluo2 HighAff AM and confocal microscopic monitoring calcium influx (Figures 1A-E). Interestingly, miR-218 overexpression increased motor neuron firing frequency, while miR-218 knockdown silenced neuronal activity (Figures 1F-G). Thus, miR-218 governs motor neuron excitability.

Consistently, miR-218 overexpression increased neuron firing frequency also in hippocampal neurons, (Figures 2A-B). Thus, miR-218 may be a broader regulator of excitability that is particularly abundant in the motor neuron system.

### EXAMPLE 2

### Unbiased target discovery reveals neuronal targets for miR-218

To experimentally identify the targets of miR-218 an advanced next generation sequencing study was performed. Total RNA was isolated from primary MNs 72 hours after viral transduction, poly-A cDNA pools created, next generation sequencing and bioinformatics analysis performed (Figure 5A). Unbiased analysis Sylamer study, that automatically depicts over- and under-representation of miRNA recognition sequences (seed-matches) [previously discussed in van Dongen, S. et al., Nat Methods (2008) 5(12): 1023-5], resulted in the identification of a cryptic miR-218 signature in the 3'URTS of differentially expressed genes (Figure 5B). Therefore, miR-218 perturbation holds a unique molecular impact (Figure 5C).

Intersecting the differentially expressed gene list with the list of predicted miR-218 targets (TargetScan algorithm previously discussed in Friedman, R.C., et al., Genome Res (2009) 19(1): 92-105) resulted in the analysis shown in Figure 5E. Genes that were downregulated following miR-218 overexpression, upregulated by miR-218 knockdown and were also predicted miR-218 targets are exemplified. A group of genes that are involved in synaptic transmission and plausibly play roles in control of neuronal excitability are identified and include potassium channels: KCNA1 (Kv1.1), KCND2 (Kv4.2) and KCNH1 (Kv10.1); the ionotropic glutamate receptor subunits GRIA2 (GluA2), GRIK2 (GluK2) and GRIK3 (GluK3); GABA receptor subunits GABRB2 and GABRG1; GABA transporters SLC6A1 (GAT1) and SLC6A11 (GAT3), as well as the calcium channel CACNB4. Several of these genes were previously found to be linked to epilepsy and are targets of clinical approved anti-seizure drugs. Several of these targets were further validated by qPCR (Figures 3-4 and 5F).

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. An agent selected from the group consisting of miR-218, miR-218*, precursor thereof and a polynucleotide sequence encoding miR-218 or miR-218* or precursor thereof, for use in the treatment of a motor neuron disease (MND).

2. The agent for use of claim 1, wherein said agent is formulated for administration into the CNS of a subject in need thereof.

3. The agent for use of claim 1, wherein the MND is selected from the group consisting of Amyotrophic Lateral Sclerosis (ALS), primary lateral sclerosis, progressive muscular atrophy, pseudobulbar palsy, progressive bulbar palsy, lower motor neuron disease, spinal muscular atrophy and epilepsy.

4. The agent for use of claim 1, wherein said MND comprises Amyotrophic Lateral Sclerosis (ALS).

5. The agent for use or article of manufacture of claim 4, wherein said ALS is an inherited ALS.

6. The agent for use or article of manufacture of claim 4, wherein said ALS is a sporadic ALS.

7. The agent for use of claim 2, wherein said subject is a human subject.

8. The agent for use of claim 1, wherein said miR-218 is as set forth in SEQ ID NO: 3.

9. The agent for use of claim 1, wherein said miR-218* is as set forth in SEQ ID NO: 4 or 5.
